# EUROPEAN PATENT SPECIFICATION

(11) **EP 2 114 933 B1**
(45) Date of publication and mention of the grant of the patent: **07.09.2011**
(21) Application number: 08702095.4
(22) Date of filing: 04.01.2008
(51) Int. Cl.: C07D 413/04, A61K 31/454, A61P 3/04, A61P 3/10

(54) **PIPERIDINE GPCR AGONISTS**
PIPERIDIN-GPCR-AGONISTEN
AGONISTES DU GPCR DE PIPÉRIDINE

(30) Priority: 04.01.2007 GB 0700123; 10.10.2007 GB 0719760
(43) Date of publication of application: 11.11.2009
(60) Divisional application: 11174256.5
(73) Proprietor: PROSIDION LTD, Oxford, OX4 6LT (GB)
(72) Inventor: FYFE, Matthew, Colin, Thor, Oxford Oxfordshire OX4 6LT (GB); KEILY, John, Oxford Oxon OX4 6LT (GB); SWAIN, Simon, Andrew, Watlington Oxfordshire OX4 6LT (GB)
(74) Representative: Talbott, Dawn Jacqueline
(86) International application number: PCT/GB2008/050010
(87) International publication number: WO 2008/081204

(56) References cited:
- WO-A-2006/067531
- WO-A-2007/003962
- WO-A-2007/116229

## Description

### BACKGROUND OF THE INVENTION

The present invention is directed to G-protein coupled receptor (GPCR) agonists. In particular, the present invention is directed to GPCR agonists that are useful for the treatment of obesity, e.g. as regulators of satiety, metabolic syndrome and for the treatment of diabetes.

Obesity is characterized by an excessive adipose tissue mass relative to body size. Clinically, body fat mass is estimated by the body mass index (BMI; weight(kg)/height(m)²), or waist circumference. Individuals are considered obese when the BMI is greater than 30 and there are established medical consequences of being overweight. It has been an accepted medical view for some time that an increased body weight, especially as a result of abdominal body fat, is associated with an increased risk for diabetes, hypertension, heart disease, and numerous other health complications, such as arthritis, stroke, gallbladder disease, muscular and respiratory problems, back pain and even certain cancers.

Pharmacological approaches to the treatment of obesity have been mainly concerned with reducing fat mass by altering the balance between energy intake and expenditure. Many studies have clearly established the link between adiposity and the brain circuitry involved in the regulation of energy homeostasis. Direct and indirect evidence suggest that serotonergic, dopaminergic, adrenergic, cholinergic, endocannabinoid, opioid, and histaminergic pathways in addition to many neuropeptide pathways (e.g. neuropeptide Y and melanocortins) are implicated in the central control of energy intake and expenditure. Hypothalamic centres are also able to sense peripheral hormones involved in the maintenance of body weight and degree of adiposity, such as insulin and leptin, and fat tissue derived peptides.

Drugs aimed at the pathophysiology associated with insulin dependent Type I diabetes and non-insulin dependent Type II diabetes have many potential side effects and do not adequately address the dyslipidaemia and hyperglycaemia in a high proportion of patients. Treatment is often focused at individual patient needs using diet, exercise, hypoglycaemic agents and insulin, but there is a continuing need for novel antidiabetic agents, particularly ones that may be better tolerated with fewer adverse effects.

Similarly, metabolic syndrome (syndrome X) places people at high risk of coronary artery disease, and is characterized by a cluster of risk factors including central obesity (excessive fat tissue in the abdominal region), glucose intolerance, high triglycerides and low HDL cholesterol, and high blood pressure. Myocardial ischemia and microvascular disease is an established morbidity associated with untreated or poorly controlled metabolic syndrome.

There is a continuing need for novel antiobesity and antidiabetic agents, particularly ones that are well tolerated with few adverse effects.

GPR119 (previously referred to as GPCR116) is a GPCR identified as SNORF25 in WO00/50562 which discloses both the human and rat receptors, US 6,468,756 also discloses the mouse receptor (accession numbers: AAN95194 (human), AAN95195 (rat) and ANN95196 (mouse)).

In humans, GPR119 is expressed in the pancreas, small intestine, colon and adipose tissue. The expression profile of the human GPR119 receptor indicates its potential utility as a target for the treatment of obesity and diabetes.

International patent applications WO2005/061489, WO2006/070208, WO2006/067531 and WO2006/067532 disclose heterocyclic derivatives as GPR119 receptor agonists. International patent applications WO2007/003960, WO2007/003961, WO2007/003962 and WO2007/003964 (published after the priority date of the present application) disclose further GPR119 receptor agonists.

The present invention relates to agonists of GPR119 which are useful for the treatment of obesity e.g. as peripheral regulators of satiety, metabolic syndrome and for the treatment of diabetes.

### SUMMARY OF THE INVENTION

Compounds of formula (I): 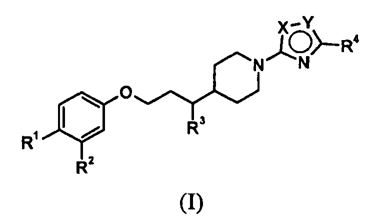 or pharmaceutically acceptable salts thereof, are agonists of GPR119 and are useful for the prophylactic or therapeutic treatment of obesity and diabetes.

### DETAILED DESCRIPTION OF THE INVENTION

The present invention is directed to a compound of formula (I), or a pharmaceutically acceptable salt thereof: 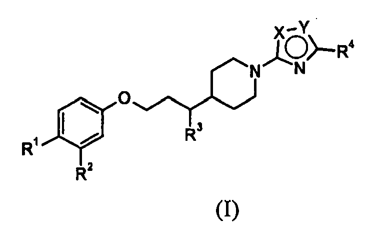
wherein one of X and Y is O and the other is N;
R¹ is SO₂R⁵;
R² is hydrogen, halo or methyl;
R³ is hydrogen or methyl;
R⁴ is C₂₋₅ alkyl; and
R⁵ is C₁₋₃alkyl.
In one embodiment of the invention X is O and in another Y is O.
R¹ is preferably -SO₂CH₃.
R² is preferably hydrogen, fluoro, chloro or methyl, more preferably hydrogen or fluoro.

In one embodiment of the invention R³ is hydrogen and in another R³ is methyl. When R³ is methyl, the stereocentre created preferably has the (*R*)-configuration.

R⁴ is preferably C₃₋₄ alkyl, particularly *n-*propyl, isopropyl or tert-butyl, more preferably C₃ alkyl, particularly isopropyl.

While the preferred groups for each variable have generally been listed above separately for each variable, preferred compounds of this invention include those in which several or each variable in formula (1) is selected from the preferred, more preferred or particularly listed groups for each variable. Therefore, this invention is intended to include all combinations of preferred, more preferred and particularly listed groups.

Specific compounds of the invention which may be mentioned are those included in the Examples and pharmaceutically acceptable salts thereof.

As used herein, unless stated otherwise, "alkyl" means carbon chains which may be linear or branched or combinations thereof. Examples of alkyl groups include methyl, ethyl, propyl, isopropyl, butyl, sec- and tert-butyl and pentyl.

The term "halo" includes fluorine, chlorine, bromine, and iodine atoms, in particular fluorine or chlorine, especially fluorine.

Compounds described herein may contain one or more asymmetric centers and may thus give rise to diastereomers and optical isomers. The present invention includes all such possible diastereomers as well as their racemic mixtures, their substantially pure resolved enantiomers, all possible geometric isomers, and pharmaceutically acceptable salts thereof. The above formula (I) is shown without a definitive stereochemistry at certain positions. The present invention includes all stereoisomers of formula (I) and pharmaceutically acceptable salts thereof. Further, mixtures of stereoisomers as well as isolated specific stereoisomers are also included. During the course of the synthetic procedures used to prepare such compounds, or in using racemization or epimerization procedures known to those skilled in the art, the products of such procedures can be a mixture of stereoisomers.

When the compound of formula (I) and pharmaceutically acceptable salts thereof exist in the form of solvates or polymorphic forms, the present invention includes any possible solvates and polymorphic forms. A type of a solvent that forms the solvate is not particularly limited so long as the solvent is pharmacologically acceptable. For example, water, ethanol, propanol, acetone or the like can be used.

The term "pharmaceutically acceptable salts" refers to salts prepared from pharmaceutically acceptable non-toxic acids, including inorganic and organic acids. Such acids include, for example, hydrochloric, methanesulfonic, sulfuric, p-toluenesulfonic acid and the like.

Since the compounds of formula (I) are intended for pharmaceutical use they are preferably provided in substantially pure form, for example at least 60% pure, more suitably at least 75% pure, especially at least 98% pure (% are on a weight for weight basis).

The compounds of formula (I) can be prepared as described below. PG represents a protecting group, G is a substituted oxadiazole as defined above, and R¹, R², R³ and R⁴ are also as defined above.

Compounds of formula (II), where PG is a suitable protecting group can be readily prepared from known compounds (Scheme 1). For example, the ethyl ester of compound (II) where PG is Boc has been previously reported (US Patent 6,518,423). Hydrogenation under standard conditions will yield the racemic compound of formula (III). Chiral reduction of the alkene under suitable conditions such as a hydrogenation in the presence of a chiral catalyst yields compounds of formula (III) in high enantiomeric excess. An example of a suitable catalyst is [Rh(norbomadiene)₂]BF₄ and (*S*)-1-[(*R*)-2-(di-*tert*-butylphosphino)ferrocenyl]-ethylbis(2-methylphenyl)phosphine. Compounds of formula (IV) can then be obtained by reduction of the carboxylic acids of formula (III) under standard conditions, for example borane in a suitable solvent such as THF. Removal of the protecting group is then achieved under conditions well known to those with skill in the art.

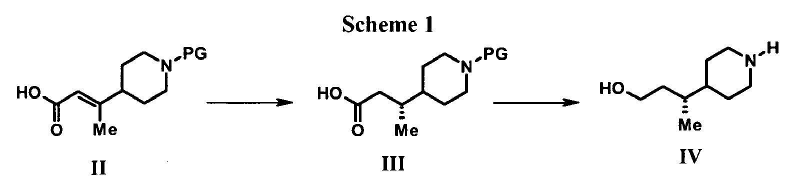

The compound of formula (V) where R³=H is a known compound (Scheme 2, Siegel, M. G. et al. Tetrahedron 1999, 55, 11619-11639). Compounds of formula (VII) can be prepared from compounds of formula (V) under standard conditions. For example, treatment of compounds of formula (V) with cyanogen bromide followed by condensation of the resultant cyanamide (VI) with a compound of formula (IX) under standard conditions yields compounds of formula (VII) where X is O. Compounds of formula (IX) are either commercially available, or readily prepared from the corresponding carboxylic acids using well known techniques. Alternatively, synthesis of the regioisomeric oxadiazole, where Y is O, can be achieved by heating compounds of formula (VI) with hydroxylamine to give *N*-hydroxyguanidines of formula (VIII) that may be condensed with a carboxylic acid of formula (X) under suitable conditions. Acids of formula (X) are commercially available.

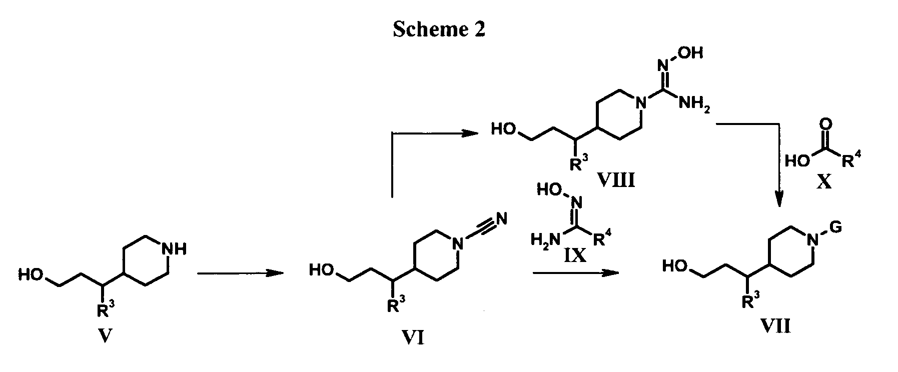

Compounds of the formula (VII) may also be prepared by condensation of amine (V) with an oxadiazole chloride of formula (XI), as illustrated in Scheme 3 (Buscemi, S. et al. JCS Perkin I: Org. and Bioorg. Chem., 1988, 1313 and Adembri, G, et al. JCS Perkin I: Org. and Bioorg. Chem., 1981, 1703).

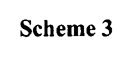

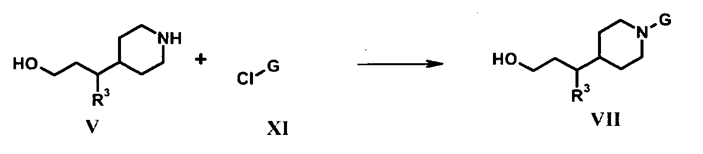

Compounds of formula (I) can be produced by combining compounds of formula (XII) and formula (VII) using Mitsunobu conditions as illustrated in Scheme 4. Compounds of formula (XII) can be readily prepared or have been previous reported (for example 3-chloro-4-methanesulfonylphenol: WO2006/049304). Furthermore, the corresponding sulfanyl precursors are also known (for example, 3-fluoro-4-methylsulfanylphenol: WO2002/083643, and is also commercially available) and can be readily oxidized to the sulfone using, for example, oxone in a suitable solvent, such as acetone, at about 0°C.

Combining compounds of formula (XII) and (VII) in a suitable solvent, such as THF, at between 0°C and room temperature, followed by the addition of triphenylphosphine and diisopropylazodicarboxylate yields the desired compounds of formula (I).

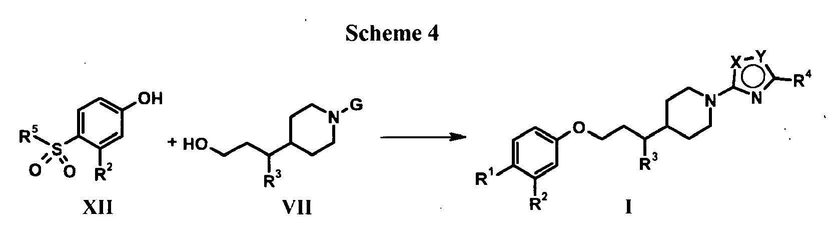

Compounds of formula (I) can also be formed as outlined in Scheme 5. In this instance, the nitrogen atom of the piperidine ring is attached to a suitable protecting group, e.g., Boc, in the alcohols of formula (Va). Mitsunobu condensation of these alcohols with phenols (XII) followed by deprotection leads to amines of formula (XIII). These amines can be converted into compounds of formula (I) using the chemistry described above in Schemes 2 and 3.

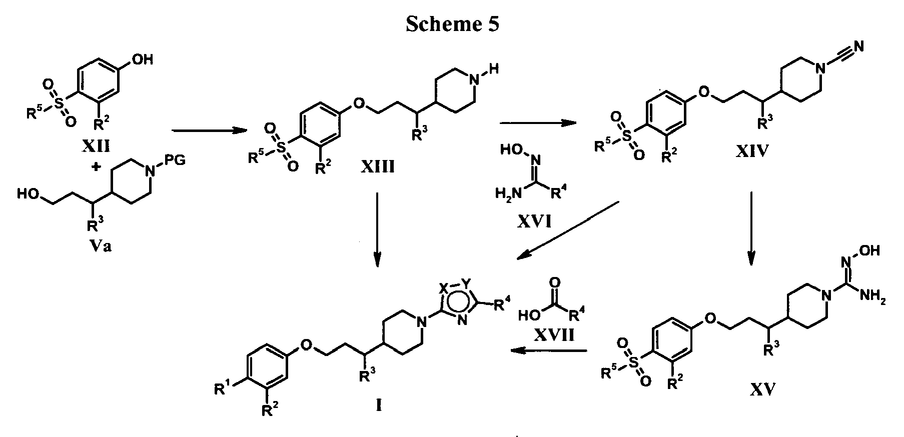

Other compounds of formula (I) may be prepared by methods analogous to those described above or by methods known *per se.*

Further details for the preparation of the compounds of formula (I) are found in the examples.

The compounds of formula (I) may be prepared singly or as compound libraries comprising at least 2, for example 5 to 1,000, compounds and more preferably 10 to 100 compounds of formula (I). Compound libraries may be prepared by a combinatorial "split and mix" approach or by multiple parallel synthesis using either solution or solid phase chemistry, using procedures known to those skilled in the art.

During the synthesis of the compounds of formula (I), labile functional groups in the intermediate compounds, e.g. hydroxy, carboxy and amino groups, may be protected. The protecting groups may be removed at any stage in the synthesis of the compounds of formula (I) or may be present on the final compound of formula (I). A comprehensive discussion of the ways in which various labile functional groups may be protected and methods for cleaving the resulting protected derivatives is given in, for example, Protective Groups in Organic Chemistry, T.W. Greene and P.G.M. Wuts, (1991) Wiley-Interscience, New York, 2nd edition.

Any novel intermediates, such as those defined above, may be of use in the synthesis of compounds of formula (I) and are therefore also included within the scope of the invention, for example compounds of formulae (XIII), (XIV) and (XV) or a salt or protected derivative thereof.

As indicated above the compounds of formula (I) are useful as GPR119 agonists, e.g. for the treatment and/or prophylaxis of obesity and diabetes. For such use the compounds of formula (I) will generally be administered in the form of a pharmaceutical composition.

The invention also provides a compound of formula (I), or a pharmaceutically acceptable salt thereof, for use as a pharmaceutical.

The invention also provides a pharmaceutical composition comprising a compound of formula (I), in combination with a pharmaceutically acceptable carrier.

Preferably the composition is comprised of a pharmaceutically acceptable carrier and a non-toxic therapeutically effective amount of a compound of formula (I), or a pharmaceutically acceptable salt thereof.

Moreover, the invention also provides a pharmaceutical composition for the treatment of disease by modulating GPR119, resulting in the prophylactic or therapeutic treatment of obesity, e.g. by regulating satiety, or for the treatment of diabetes, comprising a pharmaceutically acceptable carrier and a non-toxic therapeutically effective amount of compound of formula (I), or a pharmaceutically acceptable salt thereof.

The pharmaceutical compositions may optionally comprise other therapeutic ingredients or adjuvants. The compositions include compositions suitable for oral, rectal, topical, and parenteral (including subcutaneous, intramuscular, and intravenous) administration, although the most suitable route in any given case will depend on the particular host, and nature and severity of the conditions for which the active ingredient is being administered. The pharmaceutical compositions may be conveniently presented in unit dosage form and prepared by any of the methods well known in the art of pharmacy.

In practice, the compounds of formula (I), or pharmaceutically acceptable salts thereof, can be combined as the active ingredient in intimate admixture with a pharmaceutical carrier according to conventional pharmaceutical compounding techniques. The carrier may take a wide variety of forms depending on the form of preparation desired for administration, e.g. oral or parenteral (including intravenous).

Thus, the pharmaceutical compositions can be presented as discrete units suitable for oral administration such as capsules, cachets or tablets each containing a predetermined amount of the active ingredient. Further, the compositions can be presented as a powder, as granules, as a solution, as a suspension in an aqueous liquid, as a non-aqueous liquid, as an oil-in-water emulsion, or as a water-in-oil liquid emulsion. In addition to the common dosage forms set out above, the compound of formula (I), or a pharmaceutically acceptable salt thereof, may also be administered by controlled release means and/or delivery devices. The compositions may be prepared by any of the methods of pharmacy. In general, such methods include a step of bringing into association the active ingredient with the carrier that constitutes one or more necessary ingredients. In general, the compositions are prepared by uniformly and intimately admixing the active ingredient with liquid carriers or finely divided solid carriers or both. The product can then be conveniently shaped into the desired presentation.

The compounds of formula (I), or pharmaceutically acceptable salts thereof, can also be included in pharmaceutical compositions in combination with one or more other therapeutically active compounds.

The pharmaceutical carrier employed can be, for example, a solid, liquid, or gas. Examples of solid carriers include lactose, terra alba, sucrose, talc, gelatin, agar, pectin, acacia, magnesium stearate, and stearic acid. Examples of liquid carriers are sugar syrup, peanut oil, olive oil, and water. Examples of gaseous carriers include carbon dioxide and nitrogen.

In preparing the compositions for oral dosage form, any convenient pharmaceutical media may be employed. For example, water, glycols, oils, alcohols, flavoring agents, preservatives, coloring agents, and the like may be used to form oral liquid preparations such as suspensions, elixirs and solutions; while carriers such as starches, sugars, microcrystalline cellulose, diluents, granulating agents, lubricants, binders, disintegrating agents, and the like may be used to form oral solid preparations such as powders, capsules and tablets. Because of their ease of administration, tablets and capsules are the preferred oral dosage units whereby solid pharmaceutical carriers are employed. Optionally, tablets may be coated by standard aqueous or nonaqueous techniques.

A tablet containing the composition of this invention may be prepared by compression or molding, optionally with one or more accessory ingredients or adjuvants. Compressed tablets may be prepared by compressing, in a suitable machine, the active ingredient in a free-flowing form such as powder or granules, optionally mixed with a binder, lubricant, inert diluent, surface active or dispersing agent. Molded tablets may be made by molding in a suitable machine, a mixture of the powdered compound moistened with an inert liquid diluent. Each tablet preferably contains from about 0.05mg to about 5g of the active ingredient and each cachet or capsule preferably containing from about 0.05mg to about 5g of the active ingredient.

For example, a formulation intended for the oral administration to humans may contain from about 0.5mg to about 5g of active agent, compounded with an appropriate and convenient amount of carrier material which may vary from about 5 to about 95 percent of the total composition. Unit dosage forms will generally contain between from about 1 mg to about 2g of the active ingredient, typically 25mg, 50mg, 100mg, 200mg, 300mg, 400mg, 500mg, 600mg, 800mg, or 1000mg.

Pharmaceutical compositions of the present invention suitable for parenteral administration may be prepared as solutions or suspensions of the active compounds in water. A suitable surfactant can be included such as, for example, hydroxypropylcellulose. Dispersions can also be prepared in glycerol, liquid polyethylene glycols, and mixtures thereof in oils. Further, a preservative can be included to prevent the detrimental growth of microorganisms.

Pharmaceutical compositions of the present invention suitable for injectable use include sterile aqueous solutions or dispersions. Furthermore, the compositions can be in the form of sterile powders for the extemporaneous preparation of such sterile injectable solutions or dispersions. In all cases, the final injectable form must be sterile and must be effectively fluid for easy syringability. The pharmaceutical compositions must be stable under the conditions of manufacture and storage; thus, preferably should be preserved against the contaminating action of microorganisms such as bacteria and fungi. The carrier can be a solvent or dispersion medium containing, for example, water, ethanol, polyol (e.g. glycerol, propylene glycol and liquid polyethylene glycol), vegetable oils, and suitable mixtures thereof.

Pharmaceutical compositions of the present invention can be in a form suitable for topical use such as, for example, an aerosol, cream, ointment, lotion, dusting powder, or the like. Further, the compositions can be in a form suitable for use in transdermal devices. These formulations may be prepared, using a compound of formula (I), or a pharmaceutically acceptable salt thereof, via conventional processing methods. As an example, a cream or ointment is prepared by admixing hydrophilic material and water, together with about 5wt% to about 10wt% of the compound, to produce a cream or ointment having a desired consistency.

Pharmaceutical compositions of this invention can be in a form suitable for rectal administration wherein the carrier is a solid. It is preferable that the mixture forms unit dose suppositories. Suitable carriers include cocoa butter and other materials commonly used in the art. The suppositories may be conveniently formed by first admixing the composition with the softened or melted carrier(s) followed by chilling and shaping in molds.

In addition to the aforementioned carrier ingredients, the pharmaceutical formulations described above may include, as appropriate, one or more additional carrier ingredients such as diluents, buffers, flavoring agents, binders, surface-active agents, thickeners, lubricants, preservatives (including anti-oxidants) and the like. Furthermore, other adjuvants can be included to render the formulation isotonic with the blood of the intended recipient. Compositions containing a compound of formula (I), or pharmaceutically acceptable salts thereof, may also be prepared in powder or liquid concentrate form.

Generally, dosage levels on the order of 0.01mg/kg to about 150mg/kg of body weight per day are useful in the treatment of the above-indicated conditions, or alternatively about 0.5mg to about 7g per patient per day. For example, obesity may be effectively treated by the administration of from about 0.01 to 50mg of the compound per kilogram of body weight per day, or alternatively about 0.5mg to about 3.5g per patient per day.

It is understood, however, that the specific dose level for any particular patient will depend upon a variety of factors including the age, body weight, general health, sex, diet, time of administration, route of administration, rate of excretion, drug combination and the severity of the particular disease undergoing therapy.

The compounds of formula (I) may be used in the treatment of diseases or conditions in which GPR119 plays a role.

A method for the treatment of a disease or condition in which GPR119 plays a role comprises a step of administering to a subject in need thereof an effective amount of a compound of formula (I), or a pharmaceutically acceptable salt thereof.

Diseases or conditions in which GPR119 plays a role include obesity and diabetes. In the context of the present application the treatment of obesity is intended to encompass the treatment of diseases or conditions such as obesity and other eating disorders associated with excessive food intake e.g. by reduction of appetite and body weight, maintenance of weight reduction and prevention of rebound and diabetes (including Type 1 and Type 2 diabetes, impaired glucose tolerance, insulin resistance and diabetic complications such as neuropathy, nephropathy, retinopathy, cataracts, cardiovascular complications and dyslipidaemia). And the treatment of patients who have an abnormal sensitivity to ingested fats leading to functional dyspepsia. The compounds of the invention may also be used for treating metabolic diseases such as metabolic syndrome (syndrome X), impaired glucose tolerance, hyperlipidemia, hypertriglyceridemia, hypercholesterolemia, low HDL levels and hypertension.

The compounds of the invention may offer advantages over compounds acting via different mechanisms for the treatment of the above mentioned disorders in that they may offer beta-cell protection, increased cAMP and insulin secretion and also slow gastric emptying.

The compounds of the invention may also be used for treating conditions characterised by low bone mass such asosteopenia, osteoporosis, rheumatoid arthritis, osteoarthritis, periodontal disease, alveolar bone loss, osteotomy bone loss, childhood idiopathic bone loss, Paget's disease, bone loss due to metastatic cancer, osteolytic lesions, curvature of the spine and loss of height.

A method for the regulation of satiety comprises a step of administering to a subject in need thereof an effective amount of a compound of formula (I), or a pharmaceutically acceptable salt thereof.

A method for the treatment of obesity comprises a step of administering to a subject in need thereof an effective amount of a compound of formula (I), or a pharmaceutically acceptable salt thereof.

A method for the treatment of diabetes, including Type 1 and Type 2 diabetes, particularly type 2 diabetes, comprises a step of administering to a patient in need thereof an effective amount of a compound of formula (I), or a pharmaceutically acceptable salt thereof.

A method for the treatment of metabolic syndrome (syndrome X), impaired glucose tolerance, hyperlipidemia, hypertriglyceridemia, hypercholesterolemia, low HDL levels or hypertension comprises a step of administering to a patient in need thereof an effective amount of a compound of formula (I), or a pharmaceutically acceptable salt thereof.

The invention also provides a compound of formula (I), or a pharmaceutically acceptable salt thereof, for use in the treatment of a condition as defined above.

The invention also provides the use of a compound of formula (I), or a pharmaceutically acceptable salt thereof, in the manufacture of a medicament for the treatment of a condition as defined above.

In such methods the term "treatment" includes both therapeutic and prophylactic treatment.

The compounds of formula (I) may exhibit advantageous properties compared to known GPR119 agonists, for example, the compounds may exhibit improved potency, half-life or stability, or improved solubility thus improving absorption properties and bioavailability, or other advantageous properties for compounds to be used as pharmaceuticals.

The compounds of formula (I), or pharmaceutically acceptable salts thereof, may be administered alone or in combination with one or more other therapeutically active compounds. The other therapeutically active compounds may be for the treatment of the same disease or condition as the compounds of formula (I) or a different disease or condition. The therapeutically active compounds may be administered simultaneously, sequentially or separately.

The compounds of formula (I) may be administered with other active compounds for the treatment of obesity and/or diabetes, for example insulin and insulin analogs, gastric lipase inhibitors, pancreatic lipase inhibitors, sulfonyl ureas and analogs, biguanides, α2 agonists, glitazones, PPAR-γ agonists, mixed PPAR-α/γ agonists, RXR agonists, fatty acid oxidation inhibitors, α-glucosidase inhibitors, dipeptidyl peptidase IV inhibitors, GLP-1 agonists e.g. GLP-1 analogues and mimetics, β-agonists, phosphodiesterase inhibitors, lipid lowering agents, glycogen phosphorylase inhibitors, antiobesity agents e.g. pancreatic lipase inhibitors, MCH-1 antagonists and CB-1 antagonists (or inverse agonists), amylin antagonists, lipoxygenase inhibitors, somostatin analogs, glucokinase activators, glucagon antagonists, insulin signalling agonists, PTP1B inhibitors, gluconeogenesis inhibitors, antilypolitic agents, GSK inhibitors, galanin receptor agonists, anorectic agents, CCK receptor agonists, leptin, serotonergic/dopaminergic antiobesity drugs, reuptake inhibitors e.g. sibutramine, CRF antagonists, CRF binding proteins, thyromimetic compounds, aldose reductase inhibitors, glucocorticoid receptor antagonists, NHE-1 inhibitors or sorbitol dehydrogenase inhibitors.

Combination therapy comprising the administration of a compound of formula (I), or a pharmaceutically acceptable salt thereof, and at least one other antiobesity agent represents a further aspect of the invention.

A method for the treatment of obesity in a mammal, such as a human, comprises administering an effective amount of a compound of formula (I), or a pharmaceutically acceptable salt thereof, and another antiobesity agent, to a mammal in need thereof.

The invention also provides the use of a compound of formula (I), or a pharmaceutically acceptable salt thereof, and another antiobesity agent for the treatment of obesity.

The invention also provides the use of a compound of formula (I), or a pharmaceutically acceptable salt thereof, in the manufacture of a medicament for use in combination with another antiobesity agent, for the treatment of obesity.

The compound of formula (I), or a pharmaceutically acceptable salt thereof, and the other antiobesity agent(s) may be co-administered or administered sequentially or separately.

Co-administration includes administration of a formulation which includes both the compound of formula (I), or a pharmaceutically acceptable salt thereof, and the other antiobesity agent(s), or the simultaneous or separate administration of different formulations of each agent. Where the pharmacological profiles of the compound of formula (I), or a pharmaceutically acceptable salt thereof, and the other antiobesity agent(s) allow it, coadministration of the two agents may be preferred.

The invention also provides the use of a compound of formula (I), or a pharmaceutically acceptable salt thereof, and another antiobesity agent in the manufacture of a medicament for the treatment of obesity.

The invention also provides a pharmaceutical composition comprising a compound of formula (I), or a pharmaceutically acceptable salt thereof, and another antiobesity agent, and a pharmaceutically acceptable carrier. The invention also encompasses the use of such compositions in the methods described above.

GPR119 agonists are of particular use in combination with centrally acting antiobesity agents.

The other antiobesity agent for use in the combination therapies according to this aspect of the invention is preferably a CB-1 modulator, e.g. a CB-1 antagonist or inverse agonist. Examples of CB-1 modulators include SR141716 (rimonabant) and SLV-319 ((4*S*)-(-)-3-(4-chlorophenyl)-*N*-methyl-*N*-[(4-chlorophenyl)sulfonyl]-4-phenyl-4,5-dihydro-1H-pyrazole-1-carboxamide); as well as those compounds disclosed in EP576357, EP656354, WO 03/018060, WO 03/020217, WO 03/020314, WO 03/026647, WO 03/026648, WO 03/027076, WO 03/040105, WO 03/051850, WO 03/051851, WO 03/053431, WO 03/063781, WO 03/075660, WO 03/077847, WO 03/078413, WO 03/082190, WO 03/082191, WO 03/082833, WO 03/084930, WO 03/084943, WO 03/086288, WO 03/087037, WO 03/088968, WO 04/012671, WO 04/013120, WO 04/026301, WO 04/029204, WO 04/034968, WO 04/035566, WO 04/037823 WO 04/052864, WO 04/058145, WO 04/058255, WO 04/060870, WO 04/060888, WO 04/069837, WO 04/069837, WO 04/072076, WO 04/072077, WO 04/078261 and WO 04/108728, and the references disclosed therein.

Other diseases or conditions in which GPR119 has been suggested to play a role include those described in WO 00/50562 and US 6,468,756, for example cardiovascular disorders, hypertension, respiratory disorders, gestational abnormalities, gastrointestinal disorders, immune disorders, musculoskeletal disorders, depression, phobias, anxiety, mood disorders and Alzheimer's disease.

The invention will now be described by reference to the following examples which are for illustrative purposes.

### EXAMPLES

### Materials and methods

Column chromatography was carried out on SiO₂ (40-63 mesh) unless specified otherwise. LCMS data were obtained as follows: Atlantis 3 µC₁₈ column (3.0 × 20.0 mm, flow rate = 0.85 mL/min) eluting with a H₂O-CH₃CN solution containing 0.1% HCO₂H over 6 min with UV detection at 220 nm. Gradient information: 0.0-0.3 min 100% H₂O; 0.3-4.25 min: Ramp up to 10% H₂O-90% CH₃CN; 4.25-4.4 min: Ramp up to 100% CH₃CN; 4.4-4.9 min: Hold at 100% CH₃CN; 4.9-6.0 min: Return to 100% H₂O. The mass spectra were obtained using an electrospray ionisation source in either the positive (ES⁺) or negative (ES⁻) ion modes.

Abbreviations and acronyms: Ac: Acetyl; *n-*Bu: *n-*Butyl; *t-*Bu: *tert*-Butyl; DIAD: Diisopropyl azodicarboxylate; DIPEA: *N*,*N*-Diisopropylethylamine; DMF: Dimethylformamide; EDCI: 1-(3-Dimethylaminopropyl)-3-ethylcarbodiimide hydrochloride; Et: Ethyl; h: hour(s); HOBt: 1-Hydroxybenzotriazole; 1H: Isohexane; *i*Pr: Isopropyl; *m*CPBA: 3-Chloroperoxybenzoic acid; Me: Methyl; Ph: Phenyl; RP-HPLC: Reverse phase-high performance liquid chromatography; RT: Retention time; THF: Tetrahydrofuran.

The syntheses of the following compounds have been described elsewhere: 3-*tert*-Butyl-5-chloro-[1,2,4]oxadiazole: WO 95/05368; *tert*-Butyl 4-((E)-2-ethoxycarbonyl-1-methylvinyl)-piperidine-1-carboxylate: US Patent 6,518,423; 3-Chloro-4-methanesulfonylphenol: WO2006/049304; 3-Fluoro-4-methylsulfanylphenol: WO2002/083643; *N*-Hydroxyisobutyramidine: J. Org. Chem. 2003, 68, 7316-7321; 3-Methyl-4-methylsulfonylphenol: J. Org. Chem. 2003, 68, 5388-5391; 3-Piperidin-4-yl-propan-1-ol and *tert*-butyl 4-(3-hydroxypropyl)piperidine-1-carboxylate: Tetrahedron 1999, 55, 11619-11639. All other compounds were available from commercial sources.

### Preparation 1: 3-Fluoro-4-methanesulfonylphenol

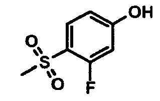

A stirred solution of 3-fluoro-4-methylsulfanylphenol (5.77 g, 36.4 mmol) in Me₂CO (37 mL) at 0°C was treated with a solution of oxone (49.31 g, 80.2 mmol) in H₂O (37 mL). After 24 h, the reaction mixture was partitioned between EtOAc and H₂O. The organic phase was washed with brine, before being dried (MgSO₄). Filtration and solvent removal yielded the title compound: δ_{H} (CDCl₃) 3.16 (s, 3H), 6.30-6.45 (br s, 1H), 6.60-6.70 (m, 2H), 7.74 (t, 1H).

### Preparation 2: 4-(3-Hydroxypropyl)piperidine-1-carbonitrile

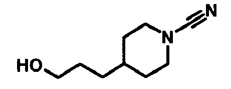

A slurry of NaHCO₃ (35.2 g, 0.42 mol) in H₂O (70 mL) was added to a stirred solution of 3-piperidin-4-ylpropan-1-ol (20.0 g, 0.14 mol) in CH₂Cl₂ at 0°C. A solution of BrCN (17.8 g, 0.17 mol) in CH₂Cl₂ (19 mL) was added to the reaction over 1 min, then stirring was continued at 0°C for 0.5 h. The reaction was then stirred at 20°C for 2 h, before being washed with saturated aqueous NaHCO₃ and brine. The CH₂Cl₂ solution was dried (MgSO₄), filtered and concentrated in vacuo to furnish an oil that was dissolved in a small amount of CH₂Cl₂, before being filtered through a SiO₂ pad, eluting with EtOAc. The filtrate was concentrated under reduced pressure to afford the title compound: *m*/*z* (ES⁺) = 169.1 [*M* + H]⁺.

### Preparation 3: N-Hydroxy-4-(3-hydroxypropyl)piperidine-1-carboxamidine

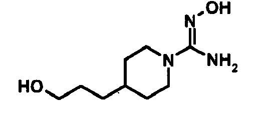

A mixture of 4-(3-hydroxypropyl)piperidine-1-carbonitrile (**Preparation 2,** 3.00 g, 17.8 mmol), K₂CO₃ (2.46 g, 17.8 mmol), and H₂NOH·HCl (2.48 g, 35.7 mmol) in EtOH (20 mL) and H₂O (30 mL) was heated under reflux for 16 h. The EtOH was removed in vacuo, then the aqueous phase was extracted with EtOAc (5x). The aqueous phase was then saturated with NaCl, before being extracted again with EtOAc (5x). The combined organic extracts were washed with brine, before being dried (MgSO₄), filtered, and concentrated to furnish the title compound: *m*/*z* (ES⁺) = 202.1 [*M*+ H]⁺.

### Preparation 4: 3-[1-(5-Isopropyl-[1,2,4]oxadiazol-3-yl)piperidin-4-yl]propan-1-ol

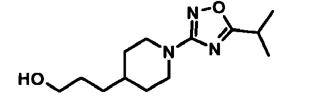

DIPEA (3.25 g, 25.2 mmol), *N*-hydroxy-4-(3-hydroxypropyl)piperidine-1-carboxamidine (**Preparation 3**, 1.54 g, 7.6 mmol), and HOBt (1.29 g, 8.4 mmol) were added to a stirred solution of isobutyric acid (0.67 g, 7.6 mmol) in anhydrous DMF (10 mL). After 10 min, EDCI (1.76 g, 9.2 mmol) was added, then stirring was continued for 16 h. The reaction was diluted with H₂O, then the mixture was extracted with EtOAc (2x). The combined organic extracts were washed with saturated aqueous NaHCO₃, H₂O, and brine, before being dried (MgSO₄). Filtration and solvent evaporation furnished a yellow oil that was treated with PhMe. The mixture was heated under reflux for 0.5 h. On cooling, the reaction was purified by column chromatography (1H-EtOAc, 2:3) to yield the title compound: *m*/*z* (ES⁺) = 254.1 [*M* + H]⁺.

### Preparation 5: 3-[1-(5-Propyl-[1,2,4]oxadiazol-3-yl)piperidin-4-yl]propan-1-ol

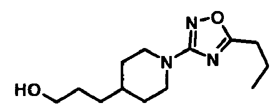

This compound was prepared from *N*-hydroxy-4-(3-hydroxypropyl)piperidine-1-carboxamidine (**Preparation 3**) and butyric acid using a procedure similar to that outlined in **Preparation 4**: *m*/*z* (ES⁺) = 254.1 [*M* + H]⁺.

### Preparation 6: 3-[1-(3-Isopropyl-[1,2,4]oxadiazol-5-yl)piperidin-4-yl]propan-1-ol

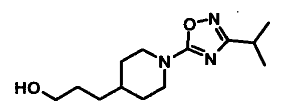

ZnCl₂ (1M in Et₂O, 145 mL, 145 mmol) was added over 20 min to a stirred solution of 4-(3-hydroxypropyl)piperidine-1-carbonitrile (**Preparation 2**, 20.3 g, 121 mmol) and *N-*hydroxyisobutyramidine (14.8 g, 145 mmol) in EtOAc (290 mL) and THF (270 mL). After 2 h, the white precipitate that had formed was collected and washed with THF-EtOAc (1:1, 50 mL). This precipitate was dissolved in EtOH (550 mL) and 12M HCl (70 mL), then the solution was stirred with heating to 70°C for 16 h. The EtOH was removed in vacuo, the remainder was diluted with H₂O, then the pH was adjusted to 7 with solid NaHCO₃. The mixture was extracted with EtOAc (3x), then the combined extracts were washed with brine, before being dried

(MgSO₄). Filtration and solvent removal furnished the title compound: *m*/*z* (ES⁺) = 254.1 [*M* + H]⁺.

### Preparation 7: 3-[1-(3-Propyl-[1,2,4]oxadiazol-5-yl)piperidin-4-yl]propan-1-ol

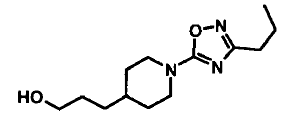

This compound was prepared by condensing 4-(3-hydroxypropyl)piperidine-1-carbonitrile (**Preparation 2**) with *N*-hydroxy-butyramidine using a procedure similar to that outlined in **Preparation 6:** *m*/*z* (ES⁺) = 254.2 [*M* + H]⁺.

### Preparation 8: tert-Butyl 4-((E)-2-carboxy-1-methylvinyl)piperidine-1-carboxylate

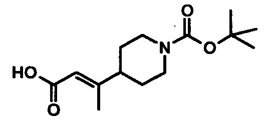

A solution of *tert*-butyl 4-((E)-2-ethoxycarbonyl-1-methylvinyl)piperidine-1-carboxylate (18.7 g, 62.9 mmol) in MeOH (90 mL) and H₂O (25 mL) was treated with 2M NaOH (94.5 mL, 189.0 mmol). The reaction was stirred for 16 h, the MeOH was removed under reduced pressure, then the remainder was partitioned between EtOAc and H₂O. The aqueous layer was separated and acidified to pH 2 with 12M HCl, before being extracted with EtOAc (2x). The organic extracts were washed with brine, dried (MgSO₄), filtered, and concentrated, then the remainder was recrystallised from EtOAc-1H to provide the title compound: *m*/*z* (ES⁻) = 268.3 [*M* - H]⁻.

### Preparation 9: tert-Butyl 4-((R)-2-carboxy-1-methylethyl)piperidine-1-carboxylate

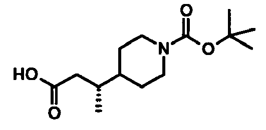

*tert*-Butyl 4-((E)-2-carboxy-1-methylvinyl)piperidine-1-carboxylate (**Preparation 8,** 130.0 g, 0.483 mol) was placed in a hydrogenation flask under an Ar atmosphere, then degassed MeOH (400 mL) was added. [Rh(norbornadiene)₂]BF₄ (1.80 g, 4.81 mmol) and (S)-1-[(R)-2-(di-*tert*-butylphosphino)ferrocenyl]ethylbis(2-methylphenyl)phosphine (2.90 g, 5.08 mmol) were placed in a separate Schlenk flask under Ar, before being treated with degassed MeOH (200 mL). This catalyst mixture was stirred for 15 min at ambient temperature, before being transferred via cannula into the hydrogenation flask. The Schlenk flask was rinsed with more degassed MeOH (100 mL). These washings were transferred to the hydrogenation flask, then more degassed MeOH (300 mL) was added. The hydrogenation flask was sealed, the Ar replaced by H₂, and the pressure set to 1.05 bar. The reaction mixture was heated to 35°C, and stirring/shaking was started. After 48 h, the reaction was stopped and a representative sample of the reaction mixture was analysed by HPLC and ¹H NMR. The conversion was 100% and the enantiomeric purity of the crude (R)-acid was 98.2%, as ascertained by the following HPLC method: Column: CHIRALPAK AD-H (previously used with CF₃CO₂H-containing solvents) 4.6 × 250 mm; Solvent: C₆H₁₄-*i*PrOH (97:3 isocratic); Temperature: 20°C; Flow rate: 1 mL/min; UV-detection (210, 230 nm); Sample: 100 µL reaction solution dissolved with 1 mL MeOH. Retention times: (S)-acid: 19.3 min, (R)-acid: 20.6 min, starting enoic acid: 22.1 min. Isolation procedure: The MeOH was evaporated, then the crude hydrogenation product was dissolved in *t-*BuOMe and extracted with aqueous NaOH. The aqueous phase was added to a mixture of 1M HCl and EtOAc. The aqueous phase was extracted further with EtOAc, then the combined organic extracts were washed with brine and dried (MgSO₄). The title compound was isolated following filtration and complete removal of the solvent.

### Preparation 10: tert-Butyl 4-((R)-3-hydroxy-1-methylpropyl)piperidine-1-carboxylate

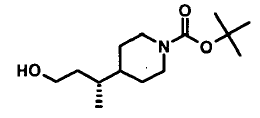

BH₃·THF (1M, 15.7 mL, 15.7 mmol) was added dropwise over 5 min to a stirred solution of *tert*-butyl 4-((R)-2-carboxy-1-methylethyl)piperidine-1-carboxylate (**Preparation 9**, 1.70 g, 6.3 mmol) in anhydrous THF at 0°C. After 1h, the reaction was treated with Et₂O, then with 2M HCl. The organic layer was washed with brine, before being dried (Na₂SO₄). Filtration, solvent evaporation, and column chromatography (EtOAc-CH₂Cl₂, 1:3) provided the title compound: RT = 3.17 min; *mlz* (ES⁺) = 258.1 [*M* + H]⁺.

### Preparation 11: 4-((R)-3-Hydroxy-1-methylpropyl)piperidine-1-carbonitrile

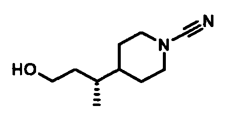

A mixture of *tert*-butyl 4-((R)-3-hydroxy-1-methylpropyl)piperidine-1-carboxylate (**Preparation 10**, 6.2 g, 14.9 mmol) and 4M HCl in dioxane (10 mL) were stirred at ambient temperature. After 3 h, the solvents were removed under reduced pressure to furnish the hydrochloride salt of (R)-3-piperidin-4-ylbutan-1-ol: δ_{H} ({CD₃}₂SO) 0.83 (d, 3H), 1.19-1.28 (m, 1H), 1.38-1.59 (m, 5H), 1.64-1.76 (m, 2H), 2.75-2.87 (m, 2H), 3.20-3.30 (m, 2H), 3.35-3.60 (m, 4H). A stirred mixture of this compound (0.93 g, 4.8 mmol) and NaHCO₃ (1.61 g, 19.2 mmol) in CH₂Cl₂-H₂O (4:1, 15 mL) at 0°C was treated with a solution of BrCN (0.61 g, 5.8 mmol) in CH₂Cl₂ (2 mL). The reaction was stirred at 20°C for 2 h, before being partitioned between H₂O and CH₂Cl₂. The organic phase was separated and dried (MgSO₄). Filtration, solvent evaporation, and flash chromatography (EtOAc) provided the title compound: RT = 2.45 min; *m*/*z* (ES⁺) = 183.1 [*M* + H]⁺.

### Preparation 12: (R)-3-[1-(3-Isopropyl-[1,2,4]oxadiazol-5-yl)piperidin-4-yl]butan-1-ol

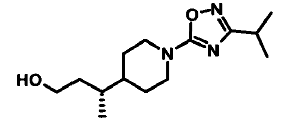

Condensation of 4-((R)-3-hydroxy-1-methylpropyl)piperidine-1-carbonitrile (**Preparation 11, 0.53** g, 2.9 mmol) with *N*-hydroxyisobutyramidine (0.36 g, 3.5 mmol), employing procedures similar to those outlined in **Preparation 6,** afforded the title compound: RT = 2.92 min; *mlz* (ES⁺) = 268.1 [*M* + H]⁺.

### Preparation 13: (R)-3-[1-(5-Isopropyl-[1,2,4]oxadiazol-3-yl)piperidin-4-yl]butan-1-ol

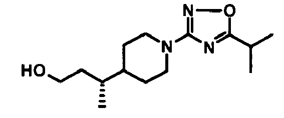

A mixture of 4-((R)-3-hydroxy-1-methylpropyl)piperidine-1-carbonitrile (**Preparation 11,** 1.00 g, 5.2 mmol) and NH₂OH (50 wt% in H₂O, 0.63 mL, 10.4 mmol) in EtOH (10 mL) was stirred at ambient temperature for 30 min. The reaction was concentrated, azeotroping with PhMe (3x), to furnish a viscous, pale yellow, oil. A mixture of this oil, EDCl (1.20 g, 6.22 mmol), HOBt (0.77 g, 5.70 mmol), isobutyric acid (0.50 mL, 5.44 mmol), and DIPEA (2.70 mL, 15.54 mmol) in anhydrous DMF (10 mL) was stirred for 16 h. The reaction was partitioned between H₂O and EtOAc. The organic layer was washed with saturated aqueous NaHCO₃ and brine, before being dried (MgSO₄), filtered, and concentrated. The remainder was heated under reflux in PhMe for 3 h, then the solvents were removed in vacuo and the residue purified by flash chromatography (EtOAc-CH₂Cl₂, 2:3) to afford the title compound: RT = 3.20 min; *m*/*z* (ES⁺) = 268.1 [*M*+H]⁺.

### Preparation 14: tert-Butyl 4-[3-(3-fluoro-4-methanesulfonylphenoxy)propyl]piperidine-1-carboxylate

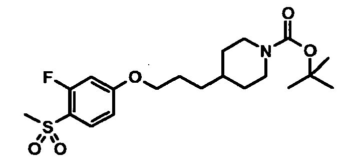

Mitsunobu condensation of 3-fluoro-4-methylsulfanylphenol with tet-t-butyl 4-(3-hydroxypropyl)piperidine-1-carboxylate, employing a procedure similar to that outlined in **Example 1,** gave teit-butyl 4-[3-(3-fluoro-4-methylsulfanylphenoxy)propyl]piperidine-1-carboxylate: *m*/*z* (ES⁺) = 384.2 *[M +* H]⁺. A stirred solution of this thioether (600 mg, 1.56 mmol) in CH₂Cl₂ (10 mL) was treated with a solution of *m*CPBA (77% pure, 720 mg, 3.21 mmol) in CH₂Cl₂ (5 mL). After 40 min, the reaction was concentrated and the remainder dissolved in EtOAc. This solution was washed with saturated aqueous Na₂CO₃ (2×), H₂O, and brine, before being dried (MgSO₄), Filtration, solvent evaporation, and column chromatography (IH-EtOAc, 2:1) afforded the title compound: RT = 3.94 min; *mlz* (ES⁺) = 416.2 [*M* + H]⁺.

### Preparation 15: 4-[3-(3-Fluoro-4-methanesulfonylphenoxy)propyl]piperidine

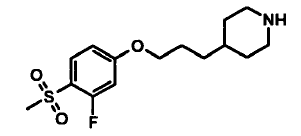

A mixture of *tert*-butyl 4-[3-(3-fluoro-4-methanesulfonylphenoxy)propyl]piperidine-1-carboxylate (**Preparation 14**, 6.2 g, 14.9 mmol) and 4M HCl in dioxane (100 mL) was stirred at ambient temperature for 1 h. The reaction was filtered and the precipitate collected was washed with Et₂O and vacuum-dried to yield the hydrochloride salt of the title compound: δ_{H} ({CD₃}₂SO) 1.17-1.29 (m, 4H), 1.49-1.60 (m, 1H), 1.71-1.84 (m, 4H), 2.74-2.87 (m, 2H), 3.17-3.26 (m, 5H), 3.45-3.60 (br, 2H), 4.03-4.10 (m, 2H), 6.96 (dd, 1H), 7.12 (dd, 1H), 7.75 (t, 1H).

### Preparation 16: 4-[3-(3-Fluoro-4-methanesulfonylphenoxy)propyl]piperidine-1-carbonitrile

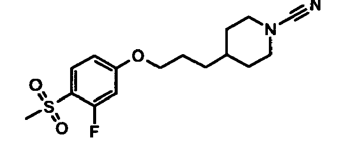

The free base of 4-[3-(3-fluoro-4-methanesulfonylphenoxy)propyl]piperidine (**Preparation 15**) was obtained by normal extractive work-up procedures. A solution of this compound (2.3 mmol) in CH₂Cl₂ (3 mL) was added to a stirred solution of NaHCO₃ (390 mg, 4.6 mmol) in H₂O (1.5 mL) at 0°C. The reaction was then treated dropwise with a solution of BrCN (29.6 mg, 2.8 mmol) in CH₂Cl₂ (4 mL). The mixture was then stirred at 0°C for 0.5 h, and then at 20°C for 1 h. The CH₂Cl₂ layer was washed with saturated aqueous NaHCO₃ and brine, before being dried (MgSO₄). Filtration, solvent removal, and column chromatography (1H-EtOAc, 1: 1) yielded the title compound: *m*/*z* (ES⁺) = 341.0 [*M* + H]⁺.

### Preparation 17: 4-[3-(3-Fluoro-4-methanesulfonylphenoxy)propyl]-N-hydroxypiperidine-1-carboxamidine

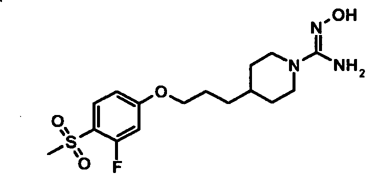

A solution of K₂CO₃ (162 mg, 1.18 mmol) and NH₂OH·HCl (162 mg, 2.35 mmol) in H₂O (5 mL) was added to a stirred suspension of 4-[3-(3-fluoro-4-methanesulfonylphenoxy)-propyl]piperidine-1-carbonitrile (**Preparation 16,** 400 mg, 1.18 mmol) in EtOH (9 mL). The reaction was heated under reflux for 16 h, then the EtOH was removed under reduced pressure. The aqueous phase was extracted with EtOAc (5×), then the combined organic extracts were washed with brine and dried (MgSO₄). Filtration and solvent evaporation yielded the title compound: *mlz* (ES⁺) = 374.0 [*M* + H]⁺.

### Preparation 18: 4-Ethanesulfonyl-3-fluorophenol

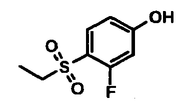

NaSEt (9.12 g, 108.4 mmol) was added over 45 min to a stirred solution of 3,4-difluoronitrobenzene (10.0 mL, 90.3 mmol) in anhydrous sulfolane (30 mL) at 0°C. The reaction was stirred at 0°C for 1 h, then at 20°C for a further 1 h, before being partitioned between Et₂O (200 mL) and H₂O (300 mL). The aqueous phase was further extracted with Et₂O (50 mL), then the combined organic extracts were washed with H₂O (5 × 100 mL) and dried (MgSO₄). Filtration, solvent evaporation, and trituration with 1H furnished 1-ethylsulfanyl-2-fluoro-4-nitrobenzene: δ_{H} (CDCl₃) 1.43 (t, 3H), 3.09 (q, 2H), 7.39 (t, 1H), 7.93 (dd, 1H), 8.02 (dd, 1H). Fe (13.32 g, 238 mmol) was added to a stirred solution of this nitrobenzene (8.00 g, 39.8 mmol) in AcOH-H₂O (84 mL, 5:1) at 0°C. After 10 min, the reaction was warmed to ambient temperature, where it was stirred for 75 min. The solvents were removed in vacuo, then the remainder was treated with 2M NaOH (150 mL), EtOAc (150 mL), and H₂O (100 mL). The organic phase was separated, then the aqueous phase was extracted further with EtOAc (200 mL). The combined organic extracts were washed with H₂O (2 × 100 mL), saturated aqueous NaHCO₃ (50 mL), and brine (50 mL), before being dried (MgSO₄), filtered and concentrated to afford 4-ethylsulfanyl-3-fluoro-phenylamine: δ_{H} (CDCl₃) 1.25 (t, 3H), 2.80 (q, 2H), 3.75-3.90 (br s, 2H), 6.38-6.43 (m, 2H), 7.22 (t, 1H). A solution of this aniline (6.53 g, 38.1 mmol) in THF (6 mL) was added to a mixture of H₂SO₄ (4 mL) and ice water (40 g) over 20 min with vigourous stirring. The reaction was stirred vigourously for a further 10 min at 0°C, then a solution of NaNO₂ (2.63 g, 38.1 mmol) in H₂O (8 mL) was added over 20 min. After being vigourously stirred for 30 min, the diazonium salt mixture produced was added, via a dropping funnel over 30 min, to a solution of CuSO₄·H₂O (21.2 g, 133.3 mmol) in H₂O (120 mL) and H₂SO₄ (11 mL), which was being heated under reflux. The reaction was heated under reflux for a further 15 min, before being allowed to cool to room temperature. The mixture was extracted with Et₂O (600 + 300 mL), then the combined organic extracts were back-extracted with 2M NaOH (300 + 100 mL). The basic aqueous extracts were acidified with 5M HCl and extracted with Et₂O (300 mL + 100 mL). The combined ethereal extracts were washed with brine (100 mL), before being dried (MgSO₄). Filtration and solvent evaporation afforded 4-ethylsulfanyl-3-fluoro-phenol: δ_{H} ({CD₃}₂SO) 1.15 (t, 3H), 2.77 (q, 2H), 6.63-6.70 (m, 2H), 7.50 (t, 1H), 10.22 (s, 1H). Oxidation of this thioether with oxone, using the procedure outlined in **Preparation 1,** provided the title compound: δ_{H} (CDCl₃) 1.25 (t, 3H), 3.32 (q, 2H), 6.72-6.81 (m, 2H), 7.80 (t, 1H).

The compounds catalogued in **Table 1** were prepared by Mitsunobu reactions, employing protocols similar to those outlined in **Example 1,** of the appropriate alcohol with either the appropriate sulfone-bearing phenol or the appropriate thioether-bearing phenol followed by *m*CPBA oxidation to the sulfone (see **Preparation 14**).

**Table 1**

| **Prep** | **Structure** | **Name** | **Spectra** |
|---|---|---|---|
| **19** | 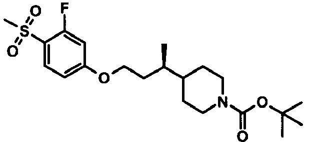 | *tert-*Butyl 4-[(R)-3-(3-fluoro-4-methanesulfonylphenoxy)-1-methylpropyl]-piperidine-1-carboxylate | RT = 4.05 min; *m*/*z* (ES⁺) = 430.1 [*M* + H]⁺ |
| **20** | 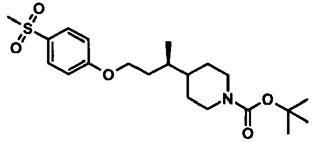 | *tert*-Butyl 4-[(R)-3-(4-methanesulfonylphenoxy)-1-methylpropyl]piperidine-1-carboxylate | RT = 3.99 min; *m*/*z* (ES⁺) = 412.1 [*M* + H]⁺ |
| **21** | 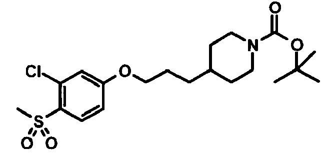 | *tert*-Butyl 4-[3-(3-chloro-4-methanesulfonylphenoxy)-propyl]piperidine-1-carboxylate | RT = 4.04 min; *m*/*z* (ES⁺) = 432.0 [*M* + H]⁺ |
| **22** | 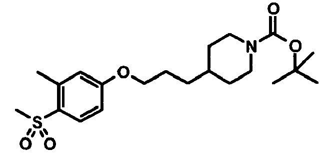 | *tert*-Butyl 4-[3-(4-methanesulfonyl-3-methylphenoxy)propyl]-piperidine-1-carboxylate | RT = 4.01 min; *m*/*z* (ES⁺) = 412.1 [*M* + H]⁺ |
| **23** | 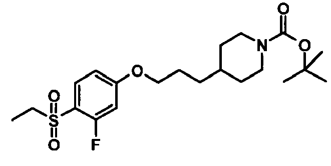 | *tert*-Butyl 4-[3-(4-ethanesulfonyl-3-fluorophenoxy)propyl]piperidine-1-carboxylate | RT = 4.01 min; *m*/*z* (ES⁺) = 430.1 [*M* + H]⁺ |

### Preparation 24: 4-[(R)-3-(3-Fluoro-4-methanesulfonylphenoxy)-1-methylpropyl]piperidine-1-carbonitrile

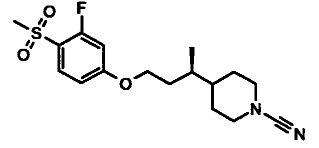

This compound was prepared from *tert*-butyl 4-[(R)-3-(3-fluoro-4-methanesulfonylphenoxy)-1-methylpropyl]piperidine-1-carboxylate (**Preparation 20**) using procedures similar to those outlined in **Preparations 15** and **16**: *mlz* (ES⁺) = 355.0 [*M* + H]⁺.

### Example 1: 4-[3-(3-Fluoro-4-methanesulfonylphenoxy)propyl]-1-(3-isopropyl-[1,2,4]oxadiazol-5-yl)piperidine

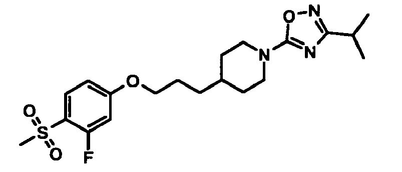

DIAD (9.4 mL, 47.8 mmol) was added dropwise to a stirred solution of 3-fluoro-4-methanesulfonylphenol (**Preparation 1**, 8.68 g, 45.6 mmol), 3-[1-(3-isopropyl-[1,2,4]oxadiazol-5-yl)piperidin-4-yl]propan-1-ol (**Preparation 6**, 11.00 g, 43.5 mmol), and PPh₃ (12.54 g, 47.8 mmol) at 0°C in anhydrous THF (350 mL). When addition was finished, stirring was continued for 90 min at 20°C, then the THF was removed under reduced pressure. The residue was dissolved in EtOAc to give a solution that was washed with 2M NaOH (2×) and brine, before being dried (MgSO₄), filtered, and concentrated in vacuo. The residue was triturated with Et₂O (4x), before being subjected to column chromatography (IH-EtOAc, 3:2 to 1:1) and a further trituration with Et₂O to yield the title compound: δ_{H} (CDCl₃) 1.27-1.39 (m, 8H), 1.45-1.63 (m, 3H), 1.80-1.94 (m, 4H), 2.93 (sept, 1H), 3.02-3.12 (m, 2H), 3.21 (s, 3H), 4.03 (t, 2H), 4.14-4.22 (m, 2H), 6.77 (dd, 1H), 6.81 (dd, 1H), 7.88 (t, 1H); *mlz* (ES⁺) = 426.3 [*M* + H]⁺.

The ethers listed in **Table 2** were synthesised through the Mitsunobu reaction, employing procedures similar to those outlined in **Example 1**.

**Table 2**

| **Ex** | **Structure** | **Name** | **Spectra** |
|---|---|---|---|
| **2** | 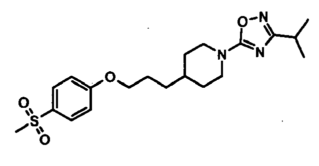 | 1-(3-Isopropyl-[1,2,4]oxadiazol-5-yl)-4-[3-(4-methanesulfonylphenoxy)-propyl]piperidine | δ_{H}(CDCl₃) 1.27-1.40 (m, 8H), 1.46-1.63 (m, 3H), 1.80-1.94 (m, 4H), 2.94 (sept, 1H), 3.02-3.12 (m, 5H), 4.05 (t, 2H), 4.17-4.22 (m, 2H), 7.02 (d, 2H), 7.90 (d, 2H); *m*/*z* (ES⁺) = 408.0 [*M* + H]⁺ |
| **3** | 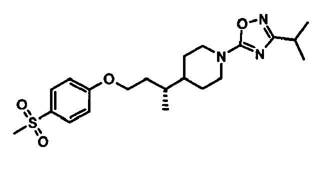 | 1-(3-Isopropyl-[1,2,4]oxadiazol-5-yl)-4-[(R)-3-(4-methanesulfonylphenoxy)-1-methylpropyl]-piperidine | δ_{H} (CDCl₃) 0.99 (d, 3H), 1.34 (d, 6H), 1.40-1.52 (m, 3H), 1.61-1.80 (m, 4H), 1.94-2.03 (m, 1H), 2.93 (sept, 1H), 2.99-3.08 (m, 5H), 4.05-4.20 (m, 2H), 4.20-4.26 (m, 2H), 7.02 (d, 2H), 7.91 (d, 2H); *m*/*z* (ES⁺) = 422.0 [*M* + H]⁺ |
| **4** | 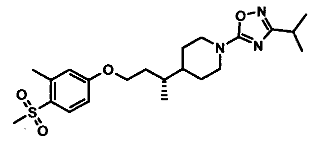 | 1-(3-Isopropyl-[1,2,4]oxadiazol-5-yl)-4-[(R)-3-(4-methanesulfonyl-3-methylphenoxy)-1-methylpropyl]piperidine | RT = 3.82 min; *m*/*z* (ES⁺) = 436.0 [*M* + H]⁺ |
| **5** | 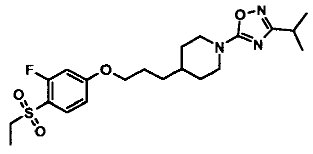 | 4-[3-(4-Ethanesulfonyl-3-fluorophenoxy)propyl]-1-(3-isopropyl- [1,2,4]oxadiazol-5-yl)piperidine | RT = 3.76 min; *m*/*z* (ES⁺) = 440.0 [*M* + H]⁺ |
| **6** | 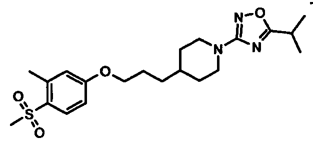 | 1-(5-Isopropyl-[1,2,4]oxadiazol-3-yl)-4-[3-(4-methanesulfonyl-3-methylphenoxy)propyl]piperidine | RT = 3.90 min; *m*/*z* (ES⁺) = 422.1 [*M* + H]⁺ |
| **7** | 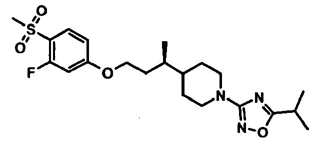 | 4-[(R)-3-(3-Fluoro-4-methanesulfonylphenoxy)-1-methylpropyl]-1-(5-isopropyl-[1,2,4]oxadiazol-3-yl)-piperidine | RT = 4.07 min; *m*/*z* (ES⁺) = 440.1 [*M* + H]⁺ |
| **8** | 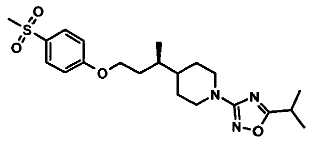 | 1-(5-Isopropyl-[1,2,4]oxadiazol-3-yl)-4-[(R)-3-(4-methanesulfonylphenoxy)-1-methylpropyl]-piperidine | RT = 3.99 min; *m*/*z* (ES⁺) = 422.1 [M + H]⁺ |
| **9** | 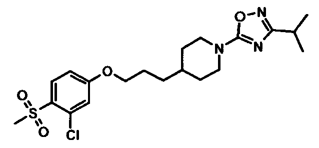 | 4-[3-(3-Chloro-4-methanesulfonylphenoxy)-propyl]-1-(3-isopropyl-[1,2,4]oxadiazol-5-yl)-piperidine | RT = 3.84 min; *m*/*z* (ES⁺) = 442.0 [*M* + H]⁺ |
| **10** | 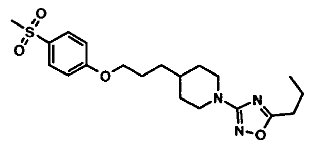 | 4-[3-(4-Methanesulfonyl-phenoxy)propyl]-1-(5-propyl-[1,2,4]oxadiazol-3-yl)-piperidine | RT = 3.79 min; *m*/*z* (ES⁺) = 408.2 [*M* + H]⁺ |
| **11** | 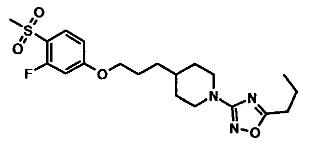 | 4-[3-(3-Fluoro-4-methanesulfonylphenoxy)-propyl]-1-(5-propyl-[1,2,4]oxadiazol-3-yl)-piperidine | RT = 3.88 min; *m*/*z* (ES⁺) = 426.3 [*M* + H]⁺ |
| **12** | 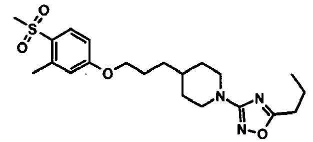 | 4-[3-(4-Methanesulfonyl-3-methylphenoxy)propyl]-1-(5-propyl-[1,2,4]oxadiazol-3-yl)-piperidine | RT = 3.87 min; *m*/*z* (ES⁺) = 422.1 [*M* + H]⁺ |
| **13** | 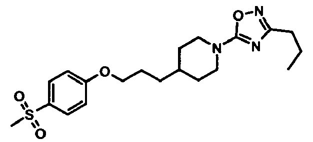 | 4-[3-(4-Methanesulfonyl-phenoxy)propyl]-1-(3-propyl-[1,2,4]oxadiazol-5-yl)-piperidine | RT = 3.64 min; *m*/*z* (ES⁺) = 408.0 [*M* + H]⁺ |
| **14** | 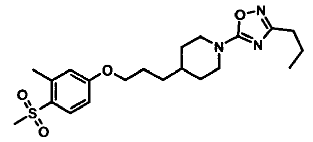 | 4-[3-(4-Methanesulfonyl-3-methylphenoxy)propyl]-1-(3-propyl-[1,2,4]oxadiazol-5-yl)-piperidine | RT = 3.77 min; *m*/*z* (ES⁺) = 422.1 [*M* + H]⁺ |
| **15** | 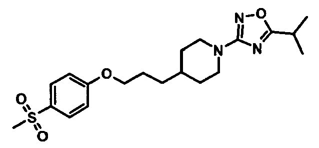 | 1-(5-Isopropyl-[1,2,4]oxadiazol-3-yl)-4-[3-(4-methanesulfonylphenoxy)-propyl]piperidine | RT = 3.77 min; *m*/*z* (ES⁺) = 408.1 [*M* + H]⁺ |
| **16** | 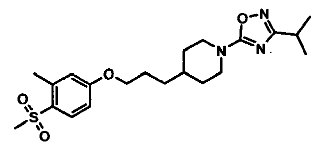 | 1-(3-Isopropyl-[1,2,4]oxadiazol-5-yl)-4-[3-(4-methanesulfonyl-3-methylphenoxy)propyl]piperidine | RT = 3.70 min; *m*/*z* (ES⁺) = 422.1 [*M* + H]⁺ |
| **17** | 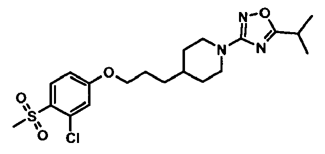 | 4-[3-(3-Chloro-4-methanesulfonylphenoxy)-propyl]-1-(5-isopropyl-[1,2,4]oxadiazol-3-yl)-piperidine | RT = 4.02 min; *m*/*z* (ES⁺) = 442.0 [*M* + H]⁺ |

### Example 18: 4-[3-(3-Fluoro-4-methanesulfonylphenoxy)propyl]-1-(3-propyl-[1,2,4]oxadiazol-5-yl)piperidine

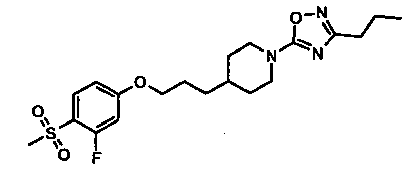

ZnCl₂ (1M in Et₂O, 0.71 mL, 0.71 mmol) was added dropwise to a stirred solution of 4-[3-(3-fluoro-4-methanesulfonylphenoxy)propyl]piperidine-1-carbonitrile (**Preparation 16**, 200 mg, 0.59 mmol) and *N*-hydroxybutyramidine (72 mg, 0.71 mmol) in EtOAc (3 mL). After 64 h, more *N*-hydroxybutyramidine (108 mg, 1.07 mmol) in EtOAc (3 mL) was added, then stirring was continued for 48 h. The mixture was concentrated, 12M HCl (0.3 mL) and EtOH (0.7 mL) were added, then the reaction was heated under reflux for 1 h. On cooling to room temperature, the reaction was neutralised with saturated aqueous NaHCO₃ and extracted twice with EtOAc. The combined EtOAc extracts were washed with brine, before being dried (MgSO₄). Filtration, solvent evaporation, column chromatography (IH-EtOAc, 1: 1), and trituration with Et₂O provided the title compound: RT = 3.64 min; *mlz* (ES⁺) = 426.0 [*M* + H]⁺.

The oxadiazoles listed in **Table 3** were synthesised by condensation of the appropriate cyanamide with the appropriate amidoxime, employing procedures similar to those outlined in **Example 18**.

**Table 3**

| **Ex** | **Structure** | **Name** | **Spectra** |
|---|---|---|---|
| **19** | 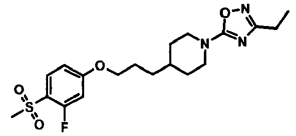 | 1-(3-Ethyl-[1,2,4]oxadiazol-5-yl)-4-[3-(3-fluoro-4-methanesulfonylphenoxy)-propyl]piperidine | RT = 3.54 min; *m*/*z* (ES⁺) = 412.1 [*M*+ H]⁺ |
| **20** | 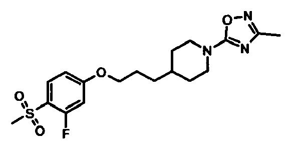 | 4-[3-(3-Fluoro-4-methanesulfonylphenoxy)-propyl]-1-(3-methyl-[1,2,4]oxadiazol-5-yl)-piperidine | RT = 3.34 min; *m*/*z* (ES⁺) = 398.0 [*M* + H]⁺ |
| **21** | 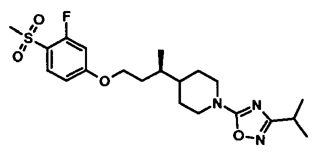 | 4-[(R)-3-(3-Fluoro-4-methanesulfonylphenoxy)-1-methylpropyl]-1-(3-isopropyl-[1,2,4]oxadiazol-5-yl)-piperidine | RT = 3.90 min; *m*/*z* (ES⁺) = 440.0 [*M* + H]⁺ |
| **22** | 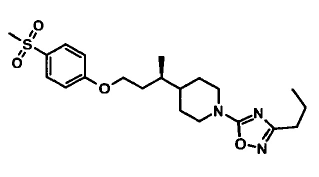 | 4-[(R)-3-(4-Methanesulfonyl-phenoxy)-1-methylpropyl]-1-(3-propyl-[1,2,4]oxadiazol-5-yl)piperidine | δ_{H} (CDCl₃) 0.98-1.04 (m, 6H), 1.38-1.81 (m, 9H), 1.96-2.03 (m, 1H), 2.57(t, 2H), 3.00-3.10 (m, 5H), 4.05-4.18 (m, 2H), 4.20-4.25 (m, 2H), 7.02 (d, 2H), 7.92 (d, 2H); *m*/*z* (ES⁺) = 422.0 [*M* + H]⁺ |
| **23** | 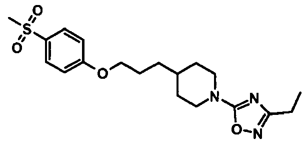 | 1-(3-Ethyl-[1,2,4]oxadiazol-5-yl)-4-[3-(4-methanesulfonyl-phenoxy)propyl]piperidine | RT = 3.39 min; *m*/*z* (ES⁺) = 394.6 [*M* + H]⁺ |

### Example 24: 1-(3-tert-Butyl-[1,2,4]oxadiazol-5-yl)-4-[3-(3-fluoro-4-methanesulfonylphenoxy)propyl]piperidine

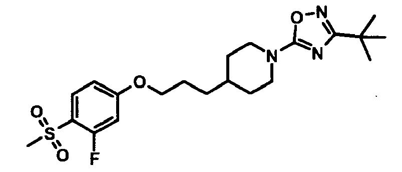

A stirred suspension of the hydrochloride salt of 4-[3-(3-fluoro-4-methanesulfonylphenoxy)propyl]piperidine (**Preparation 15**, 65 mg, 190 µmol) in anhydrous DMF (1 mL) was treated with K₂CO₃ (52 mg, 370 µmol) and a solution of 3-*tert*-butyl-5-chloro-[1,2,4]oxadiazole (30 mg, 190 µmol) in anhydrous DMF (1 mL). After 16 h, the reaction was diluted with H₂O, before being acidified with dilute HCl. The aqueous phase was extracted with Et₂O, then the combined organic extracts were dried (MgSO₄), filtered, and concentrated. The remainder was purified by flash chromatography (1H-EtOAc, 7:3) to afford the title compound: RT = 4.01 min; *mlz* (ES⁺) = 440.0 [*M* + H]⁺.

The compounds listed in **Table 4** were made from the appropriate Boc carbamate employing procedures similar to those outlined in **Preparation 15** and **Example 24**.

**Table 4**

| **Ex** | **Structure** | **Name** | **Spectra** |
|---|---|---|---|
| **25** | 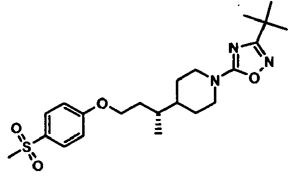 | 1-(3-*tert*-Butyl-[1,2,4]oxadiazol-5-yl)-4-[(R)-3-(4-methanesulfonylphenoxy)-1-methylpropyl]-piperidine | RT = 3.96 min; *mlz* (ES⁺) = 436.0 [*M* + H]⁺ |
| **26** | 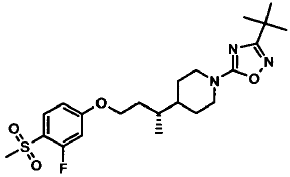 | 1-(3-*tert*-Butyl-[1,2,4]oxadiazol-5-yl)-4-[(R)-3-(3-fluoro-4-methanesulfonylphenoxy)-1-methylpropyl]-piperidine | RT = 4.01 min; *m*/*z* (ES⁺) = 454.1 [*M* + H]⁺ |
| **27** | 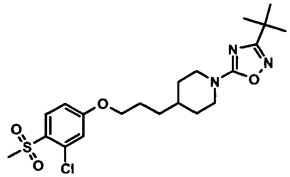 | 1-(3-*tert*-Butyl-[1,2,4]oxadiazol-5-yl)-4-[3-(3-chloro-4-methanesulfonylphenoxy)propyl]piperidine | RT = 4.02 min; *m*/*z* (ES⁺) = 456.0 [*M* + H]⁺ |
| **28** | 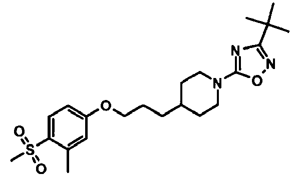 | 1-(3-*tert*-Butyl-[1,2,4]oxadiazol-5-yl)-4-[3-(4-methanesulfonyl-3-methylphenoxy)propyl]piperidine | RT = 4.12 min; *m*/*z* (ES⁺) = 436.3 [*M* + H]⁺ |
| **29** | 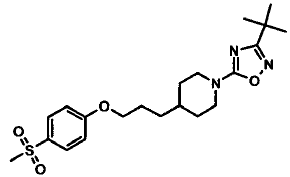 | 1-(3-*tert*-Butyl-[1,2,4]oxadiazol-5-yl)-4-[3-(4-methanesulfonylphenoxy)-propyl]piperidine | RT = 3.77 min; *m*/*z* (ES⁺) = 408.1 [*M*+ H]⁺ |
| **30** | 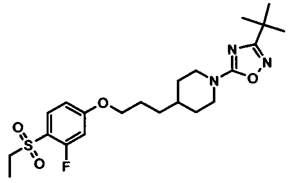 | 1-(3-*tert*-Butyl-[1,2,4]oxadiazol-5-yl)-4-[3-(4-ethanesulfonyl-3-fluorophenoxy)propyl]piperidine | RT = 4.02 min; *m*/*z* (ES⁺) = 454.1 [*M* + H]⁺ |

The compounds listed in **Table 5** were obtained by condensation of 4-[3-(3-fluoro-4-methanesulfonylphenoxy)propyl]-*N*-hydroxypiperidine-1-carboxamidine (**Preparation 17**) with the appropriate carboxylic acid, utilising procedures similar to those delineated in **Preparation 4**.

**Table 5**

| **Ex** | **Structure** | **Name** | **Spectra** |
|---|---|---|---|
| **31** | 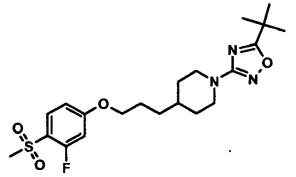 | 1-(5-*tert*-Butyl-[1,2,4]oxadiazol-3-yl)-4-[3-(3-fluoro-4-methanesulfonylphenoxy)propyl]piperidine | RT=4.11 min; *m*/*z* (ES⁺) = 440.0 [*M* + H]⁺ |
| **32** | 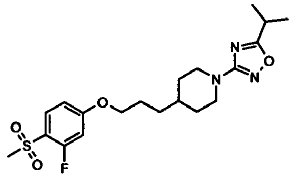 | 4-[3-(3-Fluoro-4-methanesulfonylphenoxy)-propyl]-1-(5-isopropyl-[1,2,4]oxadiazol-3-yl)-piperidine | RT = 3.82 min; *m*/*z* (ES⁺) = 426.0 [*M* + H]⁺ |
| **33** | 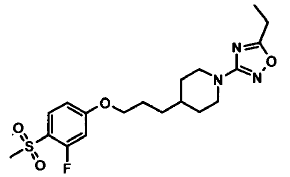 | 1-(5-Ethyl-[1,2,4]oxadiazol-3-yl)-4-[3-(3-fluoro-4-methanesulfonylphenoxy)-propyl]piperidine | RT = 3.70 min; *m*/*z* (ES⁺) = 412.1 [*M* + H]⁺ |

The biological activity of the compounds of the invention may be tested in the following assay systems:

### Yeast Reporter Assay

The yeast cell-based reporter assays have previously been described in the literature (e.g. see Miret J. J. et al, 2002, J. Biol. Chem., 277:6881-6887; Campbell R.M. et al, 1999, Bioorg. Med. Chem. Lett., 9:2413-2418; King K. et al, 1990, Science, 250:121-123); WO 99/14344; WO 00/12704; and US 6,100,042). Briefly, yeast cells have been engineered such that the endogenous yeast G-alpha (GPA1) has been deleted and replaced with G-protein chimeras constructed using multiple techniques. Additionally, the endogenous yeast GPCR, Ste3 has been deleted to allow for heterologous expression of a mammalian GPCR of choice. In the yeast, elements of the pheromone signaling transduction pathway, which are conserved in eukaryotic cells (for example, the mitogen-activated protein kinase pathway), drive the expression of Fus1. By placing β-galactosidase (LacZ) under the control of the Fus 1 promoter (Fus1p), a system has been developed whereby receptor activation leads to an enzymatic read-out.

Yeast cells were transformed by an adaptation of the lithium acetate method described by Agatep et al, (Agatep, R. et al, 1998, Transformation of Saccharomyces cerevisiae by the lithium acetate/single-stranded carrier DNA/polyethylene glycol (LiAc/ss-DNA/PEG) protocol. Technical Tips Online, Trends Journals, Elsevier). Briefly, yeast cells were grown overnight on yeast tryptone plates (YT). Carrier single-stranded DNA (10µg), 2µg of each of two Fus1p-LacZ reporter plasmids (one with URA selection marker and one with TRP), 2pg of GPR119 (human or mouse receptor) in yeast expression vector (2µg origin of replication) and a lithium acetate/ polyethylene glycol/ TE buffer was pipetted into an Eppendorf tube. The yeast expression plasmid containing the receptor/ no receptor control has a LEU marker. Yeast cells were inoculated into this mixture and the reaction proceeds at 30°C for 60min. The yeast cells were then heat-shocked at 42°C for 15min. The cells were then washed and spread on selection plates. The selection plates are synthetic defined yeast media minus LEU, URA and TRP (SD-LUT). After incubating at 30°C for 2-3 days, colonies that grow on the selection plates were then tested in the LacZ assay.

In order to perform fluorimetric enzyme assays for β-galactosidase, yeast cells carrying the human or mouse GPR119 receptor were grown overnight in liquid SD-LUT medium to an unsaturated concentration (i.e. the cells were still dividing and had not yet reached stationary phase). They were diluted in fresh medium to an optimal assay concentration and 90µl of yeast cells added to 96-well black polystyrene plates (Costar). Compounds, dissolved in DMSO and diluted in a 10% DMSO solution to 10X concentration, were added to the plates and the plates placed at 30°C for 4h. After 4h, the substrate for the β-galactosidase was added to each well. In these experiments, Fluorescein di (β-D-galactopyranoside) was used (FDG), a substrate for the enzyme that releases fluorescein, allowing a fluorimetric read-out. 20µl per well of 500µM FDG/2.5% Triton X100 was added (the detergent was necessary to render the cells permeable). After incubation of the cells with the substrate for 60min, 20µl per well of 1 M sodium carbonate was added to terminate the reaction and enhance the fluorescent signal. The plates were then read in a fluorimeter at 485/535nm.

The compounds of the invention give an increase in fluorescent signal of at least ∼ 1.5-fold that of the background signal (i.e. the signal obtained in the presence of 1% DMSO without compound). Compounds of the invention which give an increase of at least 5-fold may be preferred.

### cAMP Assay

A stable cell line expressing recombinant human GPR119 was established and this cell line was used to investigate the effect of compounds of the invention on intracellular levels of cyclic AMP (cAMP). The cell monolayers were washed with phosphate buffered saline and stimulated at 37°C for 30min with various concentrations of compound in stimulation buffer plus 1% DMSO. Cells were then lysed and cAMP content determined using the Perkin Elmer AlphaScreen^{™} (Amplified Luminescent Proximity Homogeneous Assay) cAMP kit. Buffers and assay conditions were as described in the manufacturer's protocol.

Compounds of the invention produced a concentration-dependent increase in intracellular cAMP level and generally had an EC₅₀ of < 10µM. Compounds showing and EC₅₀ of less than 1 µM in the cAMP assay may be preferred.

### In vivo feeding study

The effect of compounds of the invention on body weight and food and water intake was examined in freely-feeding male Sprague-Dawley rats maintained on reverse-phase lighting. Test compounds and reference compounds were dosed by appropriate routes of administration (e.g. intraperitoneally or orally) and measurements made over the following 24 h. Rats were individually housed in polypropylene cages with metal grid floors at a temperature of 21±4°C and 55±20% humidity. Polypropylene trays with cage pads were placed beneath each cage to detect any food spillage. Animals were maintained on a reverse phase light-dark cycle (lights off for 8 h from 09.30-17.30 h) during which time the room was illuminated by red light. Animals had free access to a standard powdered rat diet and tap water during a two week acclimatization period. The diet was contained in glass feeding jars with aluminum lids. Each lid had a 3-4 cm hole in it to allow access to the food. Animals, feeding jars and water bottles were weighed (to the nearest 0.1 g) at the onset of the dark period. The feeding jars and water bottles were subsequently measured 1, 2, 4, 6 and 24 h after animals were dosed with a compound of the invention and any significant differences between the treatment groups at baseline compared to vehicle-treated controls.

Selected compounds of the invention showed a statistically significant hypophagic effect at one or more time points at a dose of ≤ 100mg/kg.

### Anti-diabetic effects of compounds of the invention in an in-vitro model of pancreatic beta cells (HIT-T15)

### Cell Culture

HIT-T15 cells (passage 60) were obtained from ATCC, and were cultured in RPM11640 medium supplemented with 10% fetal calf serum and 30nM sodium selenite. All experiments were done with cells at less than passage 70, in accordance with the literature, which describes altered properties of this cell line at passage numbers above 81 (Zhang HJ, Walseth TF, Robertson RP. Insulin secretion and cAMP metabolism in HIT cells. Reciprocal and serial passage-dependent relationships. Diabetes. 1989 Jan;38(1):44-8).

### cAMP assay

HIT-T15 cells were plated in standard culture medium in 96-well plates at 100,000 cells/ 0.1 ml/ well and cultured for 24 hr and the medium was then discarded. Cells were incubated for 15min at room temperature with 100µl stimulation buffer (Hanks buffered salt solution, 5mM HEPES, 0.5mM 1BMX, 0.1% BSA, pH 7.4). This was discarded and replaced with compound dilutions over the range 0.001, 0.003, 0.01, 0.03, 0.1, 0.3, 1, 3, 10, 30 µM in stimulation buffer in the presence of 0.5% DMSO. Cells were incubated at room temperature for 30min. Then 75ul lysis buffer (5mM HEPES, 0.3% Tween-20, 0.1% BSA, pH 7.4) was added per well and the plate was shaken at 900 rpm for 20 min. Particulate matter was removed by centrifugation at 3000rpm for 5min, then the samples were transferred in duplicate to 384-well plates, and processed following the Perkin Elmer AlphaScreen cAMP assay kit instructions. Briefly 25µl reactions were set up containing 8µl sample, 5µl acceptor bead mix and 12µl detection mix, such that the concentration of the final reaction components is the same as stated in the kit instructions. Reactions were incubated at room temperature for 150min, and the plate was read using a Packard Fusion instrument. Measurements for cAMP were compared to a standard curve of known cAMP amounts (0.01, 0.03, 0.1, 0.3, 1, 3, 10, 30, 100, 300, 1000 nM) to convert the readings to absolute cAMP amounts. Data was analysed using XLfit 3 software.

Representative compounds of the invention were found to increase cAMP at an EC₅₀ of less than 10 µM. Compounds showing an EC₅₀ of less than 1 µM in the cAMP assay may be preferred.

### Insulin secretion assay

HIT-T15 cells were plated in standard culture medium in 12-well plates at 106 cells/ 1 ml/ well and cultured for 3 days and the medium was then discarded. Cells were washed x 2 with supplemented Krebs-Ringer buffer (KRB) containing 119 mM NaCl, 4.74 mM KCl, 2.54 mM CaCl₂, 1.19 mM MgSO₄, 1.19 mM KH2PO4, 25 mM NaHCO₃, 10mM HEPES at pH 7.4 and 0.1% bovine serum albumin. Cells were incubated with 1ml KRB at 37°C for 30 min which was then discarded. This was followed by a second incubation with KRB for 30 min, which was collected and used to measure basal insulin secretion levels for each well. Compound dilutions (0, 0.1, 0.3, 1, 3, 10 uM) were then added to duplicate wells in 1ml KRB, supplemented with 5.6 mM glucose. After 30 min incubation at 37°C samples were removed for determination of insulin levels. Measurement of insulin was done using the Mercodia Rat insulin ELISA kit, following the manufacturers instructions, with a standard curve of known insulin concentrations. For each well insulin levels were corrected by subtraction of the basal secretion level from the pre-incubation in the absence of glucose. Data was analysed using XLfit 3 software.

Representative compounds of the invention were found to increase insulin secretion at an EC₅₀ of less than 10 µM. Compounds showing an EC₅₀ of less than 1µM in the insulin secretion assay may be preferred.

### Oral Glucose Tolerance Tests

The effects of compounds of the invention on oral glucose (Glc) tolerance were evaluated in male Sprague-Dawley rats. Food was withdrawn 16 h before administration of Glc and remained withdrawn throughout the study. Rats had free access to water during the study. A cut was made to the animals' tails, then blood (1 drop) was removed for measurement of basal Glc levels 60 min before administration of the Glc load. Then, the rats were weighed and dosed orally with test compound or vehicle (20% aqueous hydroxypropyl-β-cyclodextrin) 45 min before the removal of an additional blood sample and treatment with the Glc load (2 g kg⁻¹ p.o.). Blood samples were then taken from the cut tip of the tail 5, 15, 30, 60, 120, and 180 min after Glc administration. Blood glucose levels were measured just after collection using a commercially available glucose-meter (OneTouch® UltraTM from Lifescan). Representative compounds of the invention statistically reduced the Glc excursion at doses of ≤ 10 mg kg⁻¹.

The effects of compounds of the invention on oral glucose (Glc) tolerance were also evaluated in male C57B1/6 or male *ob*/*ob* mice. Food was withdrawn 5 h before administration of Glc and remained withdrawn throughout the study. Mice had free access to water during the study. A cut was made to the animals' tails, then blood (20 µL) was removed for measurement of basal Glc levels 45 min before administration of the Glc load. Then, the mice were weighed and dosed orally with test compound or vehicle (20% aqueous hydroxypropyl-β-cyclodextrin or 25% aqueous Gelucire 44/14) 30 min before the removal of an additional blood sample (20 µL) and treatment with the Glc load (2-5 g kg⁻¹ p.o.). Blood samples (20 µL) were then taken 25, 50, 80, 120, and 180 min after Glc administration. The 20 µL blood samples for measurement of Glc levels were taken from the cut tip of the tail into disposable micro-pipettes (Dade Diagnostics Inc., Puerto Rico) and the sample added to 480 µL of haemolysis reagent. Duplicate 20 µL aliquots of the diluted haemolysed blood were then added to 180 µL of Trinders glucose reagent (Sigma enzymatic (Trinder) colorimetric method) in a 96-well assay plate. After mixing, the samples were left at rt for 30 min before being read against Glc standards (Sigma glucose/urea nitrogen combined standard set). Representative compounds of the invention statistically reduced the Glc excursion at doses ≤100 mg kg⁻¹.

## Claims

1. A compound of formula (I), or a pharmaceutically acceptable salt thereof: 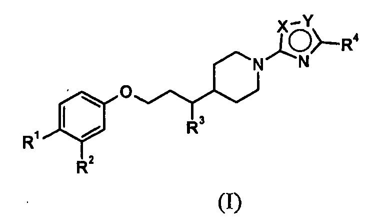
wherein one of X and Y is O and the other is N;
R¹ is SO₂R⁵;
R² is hydrogen, halo or methyl;
R³ is hydrogen or methyl;
R⁴ is C₂₋₅ alkyl; and
R⁵ is C₁₋₃ alkyl.

2. A compound according to claim 1, or a pharmaceutically acceptable salt thereof, wherein X is O.

3. A compound according to claim 1, or a pharmaceutically acceptable salt thereof, wherein Y is O.

4. A compound according to any one of claims 1 to 3, or a pharmaceutically acceptable salt thereof, wherein R¹ is SO₂CH₃.

5. A compound according to any one of claims 1 to 4, or a pharmaceutically acceptable salt thereof, wherein R² is hydrogen, fluoro, chloro or methyl.

6. A compound according to claim 5, or a pharmaceutically acceptable salt thereof, wherein R² is hydrogen or fluoro.

7. A compound according to any one of claims 1 to 6, or a pharmaceutically acceptable salt thereof, wherein R³ is hydrogen.

8. A compound according to any one of claims 1 to 6, or a pharmaceutically acceptable salt thereof, wherein R³ is methyl.

9. A compound according to claim 8, or a pharmaceutically acceptable salt thereof, wherein R³ is methyl and the stereocentre produced has the (*R*)-configuration.

10. A compound according to any one of claims 1 to 9, or a pharmaceutically acceptable salt thereof, wherein R⁴ is C₃₋₄ alkyl.

11. A compound according to claim 10, or a pharmaceutically acceptable salt thereof, wherein R⁴ is *n-*propyl, isopropyl, or *tert*-butyl.

12. A compound according to claim 11, or a pharmaceutically acceptable salt thereof, wherein R⁴ is isopropyl.

13. A compound according to claim 1, wherein said compound is 4-[3-(3-Fluoro-4-methanesulfonylphenoxy)propyl]-1-(3-isopropyl-[1,2,4]oxadiazol-5-yl)piperidine or a pharmaceutically acceptable salt thereof.

14. A compound according to claim 1, wherein said compound is 1-(3-Isopropyl-[1,2,4]oxadiazol-5-yl)-4-[(R)-3-(4-methanesulfonyl-phenoxy)-1-methylpropyl]-piperidine.

15. A compound according to claim 1 of formula (I) which is selected from 1-(3-Isopropyl-[1,2,4]oxadiazol-5-yl)-4-[3-(4-methanesulfonylphenoxy)-propyl]piperidine; 1-(3-Isopropyl-[1 ,2,4]oxadiazol-5-yl)-4-[(R)-3-(4-methanesulfonyl-3-methylphenoxy)-1-methylpropyl]piperidine; 4-[3-(4-Ethanesulfonyl-3-fluorophenoxy)propyl]-1-(3-isopropyl-[1,2,4]oxadiazol-5-yl)piperidine; 1-(5-Isopropyl-[1,2,4]oxadiazol-3-yl)-4-[3-(4-methanesulfonyl-3-methyl-phenoxy)propyl]piperidine; 4-[(R)-3-(3-Fluoro-4-methanesulfonylphenoxy)-1-methylpropyl]-1-(5-isopropyl-[1,2,4]oxadiazol-3-yl)-piperidine; 1-(5-Isopropyl-[1,2,4]oxadiazol-3-yl)-4-[(R)-3 -(4-methanesulfonyl-phenoxy)-1 -methylpropyl]-piperidine; 4-[3-(3-Chloro-4-methanesulfonylphenoxy)-propyl]-1-(3-isopropyl-[1,2,4]oxadiazol-5-yl)-piperidine; 4-[3-(4-Methanesulfonyl-phenoxy)propyl]-1-(5-propyl-[1,2,4]oxadiazol-3-yl)-piperidine; 4-[3-(3-Fluoro-4-methanesulfonylphenoxy)-propyl]-1-(5-propyl-[1,2,4]oxadiazol-3-yl)-piperidine; 4-[3-(4-Methanesulfonyl-3-methylphenoxy)propyl]-1-(5-propyl-[1,2,4]oxadiazol-3-yl)-piperidine; 4-[3-(4-Methanesulfonyl-phenoxy)propyl]-1-(3-propyl-[1,2,4]oxadiazol-5-yl)-piperidine; 4-[3-(4-Methanesulfonyl-3-methylphenoxy)propyl]-1-(3-propyl-[1,2,4]oxadiazol-5-yl)-piperidine; 1-(5-Isopropyl-[1,2,4]oxadiazol-3-yl)-4-[3-(4-methanesulfonylphenoxy)-propyl]piperidine; 1-(3-Isopropyl-[1,2,4]oxadiazol-5-yl)-4-[3-(4-methanesulfonyl-3-methyl-phenoxy)propyl]piperidine; 4-[3-(3-Chloro-4-methanesulfonylphenoxy)-propyl]-1-(5-isopropyl-[1,2,4]oxadiazol-3-yl)-piperidine; 4-[3-(3-Fluoro-4-methanesulfonylphenoxy)propyl]-1-(3-propyl-[1,2,4]oxadiazol-5-yl)piperidine; 1-(3-Ethyl-[1,2,4]oxadiazol-5-yl)-4-[3-(3-fluoro-4-methanesulfonylphenoxy)-propyl]piperidine; 4-[3-(3-Fluoro-4-methanesulfonylphenoxy)-propyl]-1-(3-methyl-[1,2,4]oxadiazol-5-yl)-piperidine; 4-[(R)-3-(3-Fluoro-4-methanesulfonylphenoxy)-1-methylpropyl]-1-(3-isopropyl-[1,2,4]oxadiazol-5-yl)-piperidine; 4-[(R)-3-(4-Methanesulfonyl-phenoxy)-1-methylpropyl]-1-(3-propyl-[1,2,4]oxadiazol-5-yl)piperidine; 1-(3-Ethyl-[1,2,4]oxadiazol-5-yl)-4-[3-(4-methanesulfonyl-phenoxy)propyl]piperidine; 1-(3-*tert*-Butyl-[1,2,4]oxadiazol-5-yl)-4-[3-(3-fluoro-4-methanesulfonyl-phenoxy)propyl]piperidine; 1-(3-*tert*-Butyl-[1,2,4]oxadiazol-5-yl)-4-[(R)-3-(4-methanesulfonyl-phenoxy)-1-methylpropyl]-piperidine; 1-(3-*tert*-Butyl-[1,2,4]oxadiazol-5-yl)-4-[(R)-3-(3-fluoro-4-methanesulfonyl-phenoxy)-1-methylpropyl]-piperidine; 1-(3-*tert*-Butyl-[1,2,4]oxadiazol-5-yl)-4-[3-(3-chloro-4-methanesulfonyl-phenoxy)propyl]piperidine; 1-(3-*tert-*Butyl-[1,2,4]oxadiazol-5-yl)-4-[3-(4-methanesulfonyl-3-methyl-phenoxy)propyl]piperidine; 1-(3-*tert*-Butyl-[1,2,4]oxadiazol-5-yl)-4-[3-(4-methanesulfonylphenoxy)-propyl]piperidine; 1-(3-tert-Butyl-[1,2,4]oxadiazol-5-yl)-4-[3-(4-ethanesulfonyl-3-fluoro-phenoxy)propyl]piperidine; 1-(5-*tert*-Butyl-[1,2,4]oxadiazol-3-yl)-4-[3-(3-fluoro-4-methanesulfonyl-phenoxy)propyl]piperidine; 4-[3-(3-Fluoro-4-methanesulfonylphenoxy)-propyl]-1-(5-isopropyl-[1,2,4]oxadiazol-3-yl)-piperidine; 1-(5-Ethyl-[1,2,4]oxadiazol-3-yl)-4-[3-(3-fluoro-4-methanesulfonylphenoxy)-propyl]piperidine; or a pharmaceutically acceptable salt thereof.

16. A pharmaceutical composition comprising a compound according to any one of claims 1 to 15, or a pharmaceutically acceptable salt thereof and a pharmaceutically acceptable carrier.

17. A compound according to any one of claims 1 to 15, or a pharmaceutically acceptable salt thereof, for use in the regulation of satiety.

18. A compound according to any one of claims 1 to 15, or a pharmaceutically acceptable salt thereof, for use in the treatment of obesity.

19. A compound according to any one of claims 1 to 15, or a pharmaceutically acceptable salt thereof, for use in the treatment of diabetes.

20. A compound according to any one of claims 1 to 15, or a pharmaceutically acceptable salt thereof, for use in the treatment of metabolic syndrome (syndrome X), impaired glucose tolerance, hyperlipidemia, hypertriglyceridemia, hypercholesterolemia, low HDL levels or hypertension.

21. A compound according to any one of claims 1 to 15, or a pharmaceutically acceptable salt thereof, for use as a medicament.

22. A compound according to any one of claims 1 to 15, or a pharmaceutically acceptable salt thereof, for use in the manufacture of a medicament for the treatment or prevention of a disease or condition as defined in any one of claims 17 to 20.

## Patentansprüche

1. Verbindung der Formel (I) oder ein pharmazeutisch annehmbares Salz davon: 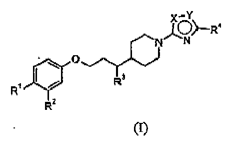
wobei eines von X und Y O ist und das andere N ist;
R¹ SO₂R⁵ ist;
R² Wasserstoff, Halogen oder Methyl ist;
R³ Wasserstoff oder Methyl ist;
R⁴ C₂₋₅-Alkyl ist; und
R⁵ C₁₋₃-Alkyl ist.

2. Verbindung nach Anspruch 1 oder ein pharmazeutisch annehmbares Salz davon, wobei X O ist.

3. Verbindung nach Anspruch 1 oder ein pharmazeutisch annehmbares Salz davon, wobei Y O ist.

4. Verbindung nach irgendeinem der Ansprüche 1 bis 3 oder ein pharmazeutisch annehmbares Salz davon, wobei R¹ SO₂CH₃ ist.

5. Verbindung nach irgendeinem der Ansprüche 1 bis 4 oder ein pharmazeutisch annehmbares Salz davon, wobei R² Wasserstoff, Fluor, Chlor oder Methyl ist.

6. Verbindung nach Anspruch 5 oder ein pharmazeutisch annehmbares Salz davon, wobei R² Wasserstoff oder Fluor ist.

7. Verbindung nach irgendeinem der Ansprüche 1 bis 6 oder ein pharmazeutisch annehmbares Salz davon, wobei R³ Wasserstoff ist.

8. Verbindung nach irgendeinem der Ansprüche 1 bis 6 oder ein pharmazeutisch annehmbares Salz davon, wobei R³ Methyl ist.

9. Verbindung nach Anspruch 8 oder ein pharmazeutisch annehmbares Salz davon, wobei R³ Methyl ist und das gebildete Stereozentrum die (*R*)-Konfiguration aufweist.

10. Verbindung nach irgendeinem der Ansprüche 1 bis 9 oder ein pharmazeutisch annehmbares Salz davon, wobei R⁴ C₃₋₄-Alkyl ist.

11. Verbindung nach Anspruch 10 oder ein pharmazeutisch annehmbares Salz davon, wobei R⁴ *n-*Propyl, Isopropyl oder *tert*-Butyl ist.

12. Verbindung nach Anspruch 11 oder ein pharmazeutisch annehmbares Salz davon, wobei R⁴ Isopropyl ist.

13. Verbindung nach Anspruch 1, wobei die Verbindung 4-[3-(3-Fluor-4-methansulfonylphenoxy)propyl]-1-(3-isopropyl-[1,2,4]oxadiazol-5-yl)piperidin oder ein pharmazeutisch annehmbares Salz davon ist.

14. Verbindung nach Anspruch 1, wobei die Verbindung 1-(3-Isopropyl-[1,2,4]oxadiazol-5-yl)-4-[(R)-3-(4-methansulfonyl-phenoxy)-1-methylpropyl]-piperidin ist.

15. Verbindung nach Anspruch 1 der Formel (I), welche ausgewählt ist aus 1-(3-Isopropyl-[1,2,4]oxadiazol-5-yl)-4-[3-(4-methansulfonylphenoxy)-propyl]piperidin; 1-(3-Isopropyl-[1,2,4]oxadiazol-5-yl)-4-[(R)-3-(4-methansulfonyl-3-methylphenoxy)-1-methyl-propyl]piperidin; 4-[3-(4-Ethansulfonyl-3-fluorphenoxy)propyl]-1-(3-isopropyl-[1,2,4]oxadiazol-5-yl)piperidin; 1-(5-Isopropyl-[1,2,4]oxadiazol-3-yl)-4-[3-(4-methansulfonyl-3-methyl-phenoxy)propyl]piperidin; 4-[(R)-3-(3-Fluor-4-methansulfonylphenoxy)-1-methylpropyl]-1-(5-isopropyl-[1,2,4]oxadiazol-3-yl)-piperidin; 1-(5-Isopropyl-[1,2,4]oxadiazol-3-yl)-4-[(R)-3-(4-methansulfonyl-phenoxy)-1-methylpropyl]-piperidin; 4-[3-(3-Chlor-4-methansulfonylphenoxy)-propyl]-1-(3-isopropyl-[1,2,4]oxadiazol-5-yl)-piperidin; 4-[3-(4-Methansulfonyl-phenoxy)propyl]-1-( -propyl-[1,2,4]oxadiazol-3-yl)-piperidin; 4-[3-(3-Fluor-4-methansulfonylphenoxy)-propyl]-1-(5-propyl-[1,2,4]oxadiazol-3-yl)-piperidin; 4-[3-(4-Methansulfonyl-3-methylphenoxy)propyl]-1-(5-propyl-[1,2,4]oxadiazol-3-yl)-piperidin; 4-[3-(4-Methansulfonyl-phenoxy)propyl]-1-(3-propyl-[1,2,4]oxadiazol-5-yl)-piperidin; 4-[3-(4-Methansulfonyl-3-methylphenoxy)propyl]-1-(3-propyl-[1,2,4]oxadiazol-5-yl)-piperidin; 1-(5-Isopropyl-[1,2,4]oxadiazol-3-yl)-4-[3-(4-methansulfonylphenoxy)-propyl]piperidin; 1-(3-Isopropyl-[1,2,4]oxadiazol-5-yl)-4-[3-(4-methansulfonyl-3-methyl-phenoxy)propyl]piperidin; 4-[3-(3-Chlor-4-methansulfonylphenoxy)-propyl]-l -(5-Isopropyl-[1,2,4]oxadiazol-3-yl)-piperidin; 4-[3-(3-Fluor-4-methansulfonylphenoxy)propyl]-1-(3-propyl-[1,2,4]oxadiazol-5-yl)piperidin; 1-(3-Ethyl-[1,2,4]oxadiazol-5-yl)-4-[3-(3-fluor-4-methansulfonylphenoxy)-propyl]piperidin; 4-[3-(3-Fluor-4-methansulfonylphenoxy)-propyl]-1-(3-methyl-[1,2,4]oxadiazol-5-yl)-piperidin; 4-[(R)-3-(3-Fluor-4-methansulfonylphonoxy)-1-methylpropyl]-1-(3-isopropyl-[1,2,4]oxadiazol-5-yl)-piperidin; 4-[(R)-3-(4-Methansulfonylphenoxy)-1-methylpropyl]-1-(3-propyl-[1,2,4]oxadiazol-5-yl)piperidin; 1-(3-Ethyl-[1,2,4]oxadiazol-5-yl)-4-[3-(4-methansulfonylphenoxy)propyl]piperidin; 1-(3-*tert*-Butyl-[1,2,4]oxadiazol-5-yl)-4-[3-(3-fluor-4-methansulfonyl-phenoxy)propyl]piperidin; 1-(3-*tert*-Butyl-[1,2,4]oxadiazol-5-yl)-4-[(R)-3-(4-methansulfonyl-phenoxy)-1-methylpropyl]-piperidin; 1-(3-tert-Butyl-[1,2,4]oxadiazol-5-yl)-4-[(R)-3-(3-fluor-4-methansulfonylphenoxy)-1-methylpropyl]-piperidin; 1-(3-*tert*-Butyl-[1,2,4]oxadiazol-5-yl)-4-[3-(3-chlor-4-methansulfonyl-phenoxy)propyl]piperidin; 1-(3-*tert*-Butyl-[1,2,4]oxadiazol-5-yl)-4-[3-(4-methansulfonyl-3-methylphenoxy)propyl]piperidin; 1-(3-*tert*-Butyl-[1,2,4]oxadiazol-5-yl)-4-[3-(4-methansulfonylphenoxy)-propyl]piperidin; 1-(3-*tert*-Butyl]-[1,2,4]oxadiazol-5-yl)-4-[3-(4-ethansulfonyl-3-fluor-phenoxy)propyl]piperidin; 1-(5-*tert*-Butyl-[1,2,4]oxadiazol-3-yl)-4-[3-(3-fluor-4-methansulfonylphenoxy)propyl]piperidin; 4-[3-(3-Fluor-4-methansulfonylphenoxy)-propyl]-1-(5-isopropyl-[1,2,4]oxadiazol-3-yl)-piperidin; 1-(5-Ethyl-[1,2,4]oxadiazol-3-yl)-4-[3-(3-fluor-4-methansulfonylphenoxy)-propyl]piperidin; oder ein pharmazeutisch annehmbares Salz davon.

16. Pharmazeutische Zusammensetzung umfassend eine Verbindung nach irgendeinem der Ansprüche 1 bis 15 oder ein pharmazeutisch annehmbares Salz davon und einen pharmazeutisch annehmbarer Träger.

17. Verbindung nach irgendeinem der Ansprüche 1 bis 15 oder ein pharmazeutisch annehmbares Salz davon zur Verwendung bei der Sättigungsregulation.

18. Verbindung nach irgendeinem der Ansprüche 1 bis 15 oder ein pharmazeutisch annehmbares Salz davon zur Verwendung bei der Behandlung von Fettleibigkeit.

19. Verbindung nach irgendeinem der Ansprüche 1 bis 15 oder ein pharmazeutisch annehmbares Salz davon zur Verwendung bei der Behandlung von Diabetes.

20. Verbindung nach irgendeinem der Ansprüche 1 bis 15 oder ein pharmazeutisch annehmbares Salz davon zur Verwendung bei der Behandlung von metabolischem Syndrom (Syndrom X), gestörter Glukosetoleranz, Hyperlipidämie, Hypertriglyzeridämie, Hypercholesterinämie, niedrigen HDL-Spiegeln oder Hypertension.

21. Verbindung nach irgendeinem der Ansprüche 1 bis 15 oder ein pharmazeutisch annehmbares Salz davon zur Verwendung als Arzneimittel.

22. Verbindung nach irgendeinem der Ansprüche 1 bis 15 oder ein pharmazeutisch annehmbares Salz davon zur Verwendung bei der Herstellung eines Arzneimittels zur Behandlung oder Vorbeugung einer in irgendeinem der Ansprüche 17 bis 20 definierten Erkrankung oder Verfassung.

## Revendications

1. Composé de formule (I), ou un sel pharmaceutiquement acceptable de celui-ci : 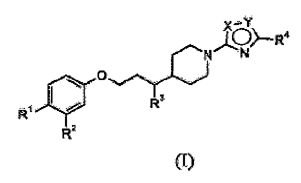
dans laquelle l'un de X et Y est O et l'autre est N ;
R¹ est SO₂R⁵ ;
R² est un hydrogène, un halogène ou un méthyle ;
R³ est un hydrogène ou un méthyle ;
R⁴ est un alkyle en C₂₋₅ ; et
R⁵ est un alkyle en C₁₋₃.

2. Composé selon la revendication 1, ou un sel pharmaceutiquement acceptable de celui-ci, dans lequel X est O.

3. Composé selon la revendication 1, ou un sel pharmaceutiquement acceptable de celui-ci, dans lequel Y est O.

4. Composé selon l'une quelconque des revendications 1 à 3, ou un sel pharmaceutiquement acceptable de celui-ci, dans lequel R¹ est SO₂CH₃.

5. Composé selon l'une quelconque des revendications 1 à 4, ou un sel pharmaceutiquement acceptable de celui-ci, dans lequel R² est un hydrogène, un Fluoro, un chloro ou un méthyle.

6. Composé selon la revendication 5, ou un sel pharmaceutiquement acceptable de celui-ci, dans lequel R² est un hydrogène ou un fluoro.

7. Composé selon l'une quelconque des revendications 1 à 6, ou un sel pharmaceutiquement acceptable de celui-ci, dans lequel R³ est un hydrogène.

8. Composé selon l'une quelconque des revendications 1 à 6, ou un sel pharmaceutiquement acceptable de celui-ci, dans lequel R³ est un méthyle.

9. Composé selon la revendication 8, ou un sel pharmaceutiquement acceptable de celui-ci, dans lequel R³ est un méthyle et le stéréocentre produit a la configuration (*R*).

10. Composé selon l'une quelconque des revendications 1 à 9, ou un sel pharmaceutiquement acceptable de celui-ci, dans lequel R⁴ est un alkyle en C₃₋₄.

11. Composé selon la revendication 10, ou un sel pharmaceutiquement acceptable de celui-ci, dans lequel R⁴ est un *n*-propyle, un isopropyle, ou un *tert-*butyle.

12. Composé selon la revendication 11, ou un sel pharmaceutiquement acceptable de celui-ci, dans lequel R⁴ est un isopropyle.

13. Composé selon la revendication 1, dans lequel ledit composé est la 4-[3-(3-fluoro-4-méthanesulfonylphénoxy)propyl]-1-(3-isopropyl-[1,2,4]oxadiazol-5-yl)pipéridine ou un sel pharmaceutiquement acceptable de celui-ci.

14. Composé selon la revendication 1, dans lequel ledit composé est la 1-(3-isopropyl-[1,2,4]oxadiazol-5-yl)-4-[(R)-3-(4-méthanesulfonyl-phénoxy)-1-méthylpropyl]-pipéridine.

15. Composé selon la revendication 1 de formule (I) qui est choisi parmi la 1-(3-isopropyl-[1,2,4]oxadiazol-5-yl)-4-[3-(4-méthanesulfonylphénoxy)-propyl]pipéridine ; 1-(3-isopropyl-[1,2,4]oxadiazol-5-yl)-4-[(R)-3-(4-méthanesulfonyl-3-méthylphénoxy)-1-méthyl-propyl]pipéridine ; 4-[3-(4-éthanesulfonyl-3-fluorophénoxy)propyl]-1-(3-isopropyl-[1,2,4]oxadiazol-5-yl)pipéridine ; 1-(5-isopropyl-[1,2,4]oxadiazol-3-yl)-4-[3-(4-méthanesulfonyl-3-méthyl-phénoxy)propyl]pipéridine ; 4-[(R)-3-(3-fluoro-4-méthanesulfonylphénoxy)-1-méthylpropyl]-1-(5-isopropyl-[1,2,4]oxadiazol-3-yl)-pipéridine ; 1-(5-isopropyl-[1,2,4]oxadiazol-3-yl)-4-[(R)-3-(4-méthanesulfonyl-phénoxy)-1-méthylpropyl]-pipéridine ; 4-[3-(3-chloro-4-méthanesulfonylphénoxy)-propyl]-1-(3-isopropyl-[1,2,4]oxadiazol-5-yl)-pipéridine ; 4-[3-(4-méthanesulfonyl-phénoxy)propyl]-1-(5-propyl-[1,2,4]oxadiazol-3-yl)-pipéridine ; 4-[3-(3-fluoro-4-méthanesulfonylphénoxy)-propyl]-1-(5-propyl-[1,2,4]oxadiazol-3-yl)-pipéridine ; 4-[3-(4-méthanesulfonyl-3-méthylphénoxy)propyl]-1-(5-propyl-[1,2,4]oxadiazol-3-yl)-pipéridine ; 4-[3-(4-méthanesulfonyl-phénoxy)propyl]-1-(3-propyl-[1,2,4]oxadiazol-5-yl)-pipéridine ; 4-[3-(4-méthanesulfonyl-3-méthylphénoxy)propyl]-1-(3-propyl-[1,2,4]oxadiazol-5-yl)-pipéridine ; 1-(5-isopropyl-[1,2,4]oxadiazol-3-yl)-4-[3-(4-méthanesulfonylphénoxy)-propyl]pipéridine ; 1-(3-isoprvpyl-[1,2,4]oxadiazol-5-yl)-4-[3-(4-méthanesulfonyl-3-méthyl-phénoxy)propyl]pipéridine ; 4-[3-(3-chloro-4-méthanesulfonylphénoxy)-propyl]-1-(5-isopropyl-[1,2,4]oxadiazol-3-yl)-pipéridine ; 4-[3-(3-fluoro-4-méthanesulfonylphénoxy)propyl]-1-(3-propyl-[1,2,4]oxadiazol-5-yl)pipéridine ; 1-(3-éthyl-[1,2,4]oxadiazol-5-yl)-4-[3-(3-fluoro-4-méthanesulfonylphénoxy)-propyl]pipéridine ; 4-[3-(3-fluoro-4-méthanesulfonylphénoxy)-propyl]-1-(3-méthyl-[1,2,4]oxadiazol-5-yl)-pipéridine ; 4-[(R)-3-(3-fluoro-4-méthanesulfonylphénoxy)-1-méthylpropyl]-1-(3-isopropyl-[1,2,4]oxadiazol-5-yl)-pipéridine ; 4-[(R)-3-(4-méthanesulfonyl-phénoxy)-1-méthylpropyl]-1-(3-propyl-[1,2,4]oxadiazol-5-yl)pipéridine ; 1-(3-éthyl-[1,2,4]oxadiazol-5-yl)-4-[3-(4-méthanesulfonyl-phénoxy)propyl]pipéridine ; 1-(3-*tert-*butyl-[1,2,4]oxadiazol-5-yl)-4-[3-(3-fluoro-4-méthanesulfonyl-phénoxy)propyl]pipéridine ; 1-(3-*tert-*butyl-[1,2,4]oxadiazol-5-yl)-4-[(R)-3-(4-méthanesulfonyl-phénoxy)-1-méthylpropyl]-pipéridine ; 1-(3-*tert-*butyl-[1,2,4]oxadiazol-5-yl)-4-[(R)-3-(3-fluoro-4-méthanesulfonyl-phénoxy)-1-méthylpropyl]-pipéridine ; 1-(3-*tert-*butyl-[1,2,4]oxadiazol-5-yl)-4-[3-(3-chloro-4-méthanesulfonyl-phénoxy)propyl]pipéridine ; 1-(3-*tert*-butyl-[1,2,4]oxadiazol-5-yl)-4-[3-(4-méthanesulfonyl-3-méthyl-phénoxy)propyl]pipéridine ; 1-(3-*tert-*butyl-[1,2,4]oxadiazol-5-yl)-4-[3-(4-méthanesulfonylphénoxy)-propyl]pipéridine ; 1-(3-*tert-*butyl-[1,2,4]oxadiazol-5-yl)-4-[3-(4-éthanesulfonyl-3-fluoro-phénoxy)propyl]pipéridine ; 1-(5-*tert-*butyl-[1,2,4]oxadiazol-3-yl)-4-[3-(3-fluoro-4-méthanesulfonyl-phénoxy)propyl]pipéridine ; 4-[3-(3-fluoro-4-méthanesulfonylphénoxy)-propyl]-1-(5-isopropyl-[1,2,4]oxadiazol-3-yl)-pipéridine ; 1-(5-éthyl-[1,2,4]oxadiazol-3-yl)-4-[3-(3-fiuoro-4-méthanesulfonylphénoxy)-propyl]pipéridine ; ou un sel pharmaceutiquement acceptable de celui-ci.

16. Composition pharmaceutique comprenant un composé selon l'une quelconque des revendications 1 à 15, ou un sel pharmaceutiquement acceptable de celui-ci et un support pharmaceutiquement acceptable.

17. Composé selon l'une quelconque des revendications 1 à 15, ou un sel pharmaceutiquement acceptable de celui-ci, pour une utilisation dans la régulation de la satiété.

18. Composé selon l'une quelconque des revendications 1 à 15, ou un sel pharmaceutiquement acceptable de celui-ci, pour une utilisation dans le traitement de l'obésité.

19. Composé selon l'une quelconque des revendications 1 à 15, ou un sel pharmaceutiquement acceptable de celui-ci, pour une utilisation dans le traitement du diabète.

20. Composé selon l'une quelconque des revendications 1 à 15, ou un sel pharmaceutiquement acceptable de celui-ci, pour une utilisation dans le traitement d'un syndrome métabolique (syndrome X), d'une tolérance au glucose altérée, d'une hyperlipidémie, d'une hypertriglycéridémie, d'une hypercholestérolémie, de faibles taux de HDL ou d'une hypertension.

21. Composé selon l'une quelconque des revendications 1 à 15, ou un sel pharmaceutiquement acceptable de celui-ci, pour une utilisation en tant que médicament.

22. Composé selon l'une quelconque des revendications 1 à 15, ou un sel pharmaceutiquement acceptable de celui-ci, pour une utilisation dans la fabrication d'un médicament destiné au traitement ou à la prévention d'une maladie ou d'un état tel que défini selon l'une quelconque des revendications 17 à 20.
